# EUROPÄISCHE PATENTANMELDUNG

(11) **EP 2 600 153 A1**
(43) Veröffentlichungstag der Anmeldung: **05.06.2013**
(21) Anmeldenummer: 12195138.8
(22) Anmeldetag: 30.11.2012
(51) Int. Cl.: G01N 33/574

(54) **Identifikation und Gewinnung von Tumorstammzellen**

(30) Priorität: 02.12.2011 EP 11009545
(71) Anmelder: Fraunhofer-Gesellschaft zur Förderung der angewandten Forschung e.V., 80686 München (DE)
(72) Erfinder: Ruschpler, Peter, 04205 Leipzig (DE); Brinckmann, Ute, 04317 Leipzig (DE); Fabian, Claire, 04107 Leipzig (DE)
(74) Vertreter: Lahrtz, Fritz

(57) **Zusammenfassung**

Die vorliegende Erfindung betrifft unter anderem Verfahren zur Detektion, Identifikation und/oder Gewinnung von Tumorstammzellen basierend auf ihrem Zellteilungsverhalten, dadurch gekennzeichnet, dass Tumorzellen in der Tumorprobe, die keine Tumorstammzellen sind und ein im Vergleich zu Tumorstammzellen schnelleres Zellteilungsverhalten aufweisen, einer Markierung unterzogen werden. Die Erfindung betrifft ferner die Verwendung der so identifizierten und gewonnenen Tumorstammzellen in der Medizin sowie mit den oben genannten Verfahren in Zusammenhang stehende Verwendungen.

## Beschreibung

### Gebiet der Erfindung

Die vorliegende Erfindung betrifft das Gebiet von (Tumor)Stammzellen. Unter anderem beschäftigt sich die vorliegende Erfindung mit der Detektion, Identifikation, Fraktionierung und Gewinnung von (Tumor)Stammzellen, beispielsweise aus einem Gewebeexplantat eines menschlichen Individuums, und setzt dabei insbesondere auf die Negativselektion solcher Zellen aufgrund ihres Zellteilungsverhaltens sowie auf die Markierung von Tumorzellen, die ein im Vergleich zu den Tumorstammzellen schnelleres Zellteilungsverhalten aufweisen.

### Hintergrund der Erfindung

Tumorstammzellen, im Fachgebiet und hierin auch als CSC (von *"cancer*/ *tumor stem cells"*) oder Tumor-initiierende Zellen (bzw. *"tumor initiating cells"*) bezeichnet, spielen eine wichtige Rolle in der Ausbildung und Aufrechterhaltung von Tumorerkrankungen.

Metastasierende Formen maligner Erkrankungen bleiben bei konventioneller Chemotherapie und Bestrahlung, bis heute sehr häufig therapieresistent. Die Inzidenz solider Tumore steigt derzeit weltweit progressiv an. Dabei werden laut Auskunft der WHO aus dem Jahr 2008 pro Jahr weltweit ca. 12 Mio. Fälle neu diagnostiziert, von denen 5-10% tödlich verlaufen. Jeder dritte Patient hat zum Zeitpunkt der Diagnose bereits Metastasen und 30-40% aller Fälle entwickeln diese nach chirurgischer Therapie (Lam JS et al., 2005). Beim hellzelligen Nierenzellkarzinom zum Beispiel, stellt eine radikale oder teilweise Nierenektomie heute den "Goldstandard" für das Management einer klinisch lokalisierten Erkrankung dar (Leibovich BC et al., 2003). Seit der Konferenz der ASCO ("American Society of Clinical Oncology") im Jahre 2007 hat zudem ein Paradigmenwechsel in der medikamentösen Behandlung stattgefunden. Sechs verschiedene Substanzen, die vor allem in die Tumorangiogenese eingreifen, sind bis heute zugelassen worden. Darunter befinden sich Sunitinib, Pazopanib, Sorafenib, Temsirolimus, Bevacizumab und Everolimus. All diese Bemühungen berücksichtigen ein wesentliches Charakteristikum der Tumorentstehung nicht oder nur ungenügend. Schon Otto Warburg konnte 1932 zeigen, dass embryonale Zellen unter Hypoxie, Eigenschaften von Tumorzellen entwickeln. Es kommt zu einem vermehrten Wachstum dieser Zellen, durch die Vergärung und von Glukose zu Laktat. Ein entsprechend stabiles Sauerstoffangebot ist also für die Tumorprogression nicht zwingend erforderlich und auch die neuen anti-angiogenetischen Medikamente greifen für sich genommen zu kurz. Ein malignes Ereignis ist also vor allem durch die Präsenz nicht mehr regulierbarer Stammzellen gekennzeichnet. Der eigentliche Schritt einer Tumorentstehung, noch vor dem Erscheinen eines Primärtumors, ist demnach die Entstehung der ersten maligne transformierten Stammzelle (tumorigene Tumorzellen) des betreffenden Gewebes.

Tatsächlich kann in einer Zusammenschau die Existenz einer Tumorstammzellpopulation durch drei wesentliche Beobachtungen definiert werden: 1. Nur eine Minorität von Tumorzellen einer Tumorentität ist mit einem "tumorigenen" Potential ausgestattet. 2. Tumorigene Tumorzellen sind durch ein bestimmtes Profil an Oberflächenmarkern charakterisiert und können aus Gesamttumoren mit verschiedenen Methoden der Immunoselektion isoliert werden. 3. Tumore wachsen ausgehend von tumorigenen Zellen (CSC) und beinhalten gemischte Populationen an tumorigenen und nicht tumorigenen Tumorzellen, die dann die volle phänotypische Heterogenität des parentalen Tumors wiederherstellen. Das Auftreten von tumorigenen Tumorzellen in einem soliden Gesamtmalignom wurde 2002 erstmals von M. Al-Hajj et al. für das Mamakarzinom nachgewiesen (Al-Hajj M et al., 2003).

Tumore sind aus heterogenen Zellpopulationen aufgebaut, die sich in ihrem jeweiligen Differenzierungsgrad unterscheiden. Tumore sind gerade nicht monoklonale Expansionen von Zellen, könnten aber ähnlich wie "abnormale" Organe durch eine "erkrankte" Tumorstammzellpopulation unterhalten werden. Diese ist ausgestattet mit der Fähigkeit zur Selbsterneuerung und unterliegt einer - für das betreffende Gewebe - abweichenden Differenzierung. CSC disseminieren, bereits sehr lange vor dem Auftreten eines Primärtumors, durch Mutationen in einzelnen entwicklungsbiologischen Pathways, wie Notch, Wnt, BMI-1 und Shh im entsprechenden Gewebe. Vor diesem Hintergrund liegt die Vermutung sehr nahe, dass es sich um veränderte Gewebsstammzellen handelt, zumal auch eine bestimmte Mutation mit einer spezifischen Tumorentität korrespondiert.

Das Tumorstammzellmodell hat die biomedizinische Beurteilung über die Ätiologie und Charakteristik eines maligne-neoplastischen Ereignisses grundlegend verändert. Während man noch vor wenigen Jahren von einer Klonalitätshypothese ausging, bei der jede neue Tumorzelle zur Bildung eines neuen Tumors in der Lage sein soll (tumorigen), weiß man heute, dass die Tumorgenese mit einem sogenannten hierarchischen Modell der Zellarchitektur beschrieben werden kann. Dabei spielen Tumorzellen mit Stammzelleigenschaften (hierin beispielsweise auch als Tumorstammzellen oder "CSC" bezeichnet), von denen sich Progenitoren, transient differenzierte-, differenzierte- und terminal differenzierte Tumorzellen ableiten, eine entscheidende Rolle. In diesem Modell, welches zuerst für die AML und das Mamakarzinom verifiziert werden konnte, sind allein die CSC tumorigen und damit in der Lage einen neuen Tumor zu indizieren. Zudem befinden sie sich in einer sogenannten Stammzellnische, sodass sie den konventionellen zytostatischen Therapien, die vor allem auf die sich schnell teilenden Tumorzellen (residuale Tumorzellen) fokussieren, auszuweichen in der Lage sind.

Bis heute wurden Tumorstammzellen beispielsweise über ihre Expansion in sogenannten Kolonie- bzw. Sphäroid-Assays gewonnen. Um Tumorstammzellen einer bestimmten Population zu vermehren, können Kulturbedingungen gewählt werden, die die Proliferation undifferenzierter Zellen begünstigen. Um die Adhäsion und die resultierende Differenzierung der Tumorstammzellen zu vermeiden, werden die Zellen in sogenannten Platten mit sehr geringer Adhäsion ("ultra-low adhesion plates") kultiviert. Die Kultivierung erfolgt zumeist in serumfrei-konditionierten Medien (z. Bsp. DMEM-F12, Invitrogen), das mit spezifischen Wachstumsfaktoren, wie FGF-23, TGF und bFGF versetzt ist. Die Zellen werden beispielsweise nach der jeweiligen Behandlung in Platten mit 6 Vertiefungen ("6-wellPlatten") in einer Konzentration von 10.000 Zellen pro ml ausgesät. Unter diesen Kulturbedingungen bilden Tumorstammzellen (beispielsweise vergleichbar mit normalen neuralen Stammzellen) dreidimensionale, frei schwimmende Zellaggregate aus, die Sphären ("spheres") genannt werden. Diese 3-D-Architektur wird durch die Zugabe von Kollagen unterstützt. Zur Quantifizierung wurden standardisiert vier Gesichtsfelder von mindestens drei Wells von zwei Personen unabhängig voneinander ausgezählt. Die Sphären enthalten nach bis zu 4 Wochen, bei einer Größe <1,5 mm, zu 100% Tumor-/Stammzellen. Auf der Basis dieser Beobachtungen konnte ein Tumorstammzellmodell entwickelt werden, das zur Beschreibung von maligne neoplastischen Ereignissen Anwendung findet. Der oben beschriebene diagnostische und therapeutische Ansatz, muss dabei um die Detektion und Elimination von tumorigenen Tumorzellen (Tumorstammzellen) erweitert werden, um die stetige Unterhaltung des Malignoms durch diese chemo- und radioresistenten Zellen zu beenden.

Einen wichtigen Befund für die molekularzytologische Beschreibung und weitere Adressierung solider Tumore lieferte die Detektion von tumorassoziierten Antigenen und Stammzellmarkern auf der Oberfläche von Zellen verschiedener Fraktionen eines Malignoms. Die Suche nach Biomarkern ist insbesondere dafür eine außerordentlich aktive Domäne der derzeitigen Forschung (Kim HL et al., 2005). Die Verbesserung der Diagnose hinsichtlich der Identifikation von "high risk" - Patienten mit hoher Rezidiv-Wahrscheinlichkeit, erlaubt ein einfachere Nachkontrolle (hierin auch als "follow-up"), verspricht eine frühere adjuvante Behandlungsmöglichkeit und damit die Selektion der bestmöglichen Behandlung für Patienten mit metastasierenden Verläufen.

Beispielsweise repräsentiert die Carboanhydrase 9 ("CA09", hierin auch "CA_09"), eines der meist untersuchten Oberflächenantigene für das Nierenzellkarzinom, derzeit einen der robustesten Biomarker mit den meistbeschriebenen Kriterien für diese Tumorentität. Carboanhydrasen (CAs) sind Metalloenzyme, die in nahezu allen Geweben exprimiert werden. Die Katalyse der reversiblen Reaktion CO₂ + H₂O ⇔ H₂CO₃ ist ihre Hauptfunktion. Bicarbonat wird bei der katalytischen Aktivität der intrazellulären CAs durch ein Proton aktiv inkorporiert und neutralisiert. Dieser Prozess generiert Kohlendioxid welches dann passiv in den extrazellulären Raum diffundiert, wo es dann von extrazellulären CAs opsoniert und wieder zu Bicarbonat und einem Proton konvertiert wird. Das Ergebnis, typisch für solide Tumore, ist eine Neutralisation des intrazellulären- bei gleichzeitiger Azidifikation des extrazellulären Milieus. Der monoklonale Antikörper (mAb) G250 ("Grawitz 250"), der ein transmembranales Antigen von Nierenkarzinomzellen (CCRCC) erkennt, welches aber nicht auf normal-gesunden Nierengewebszellen exprimiert ist, wurde bereits 1986 beschrieben (Oosterwwijk E et al., 1986; Leibovich BC et al., 2007). Dieser Antikörper bindet an CA09. Die CA09 ist in 94-97% aller Nierenkarzinome exprimiert (Patard JJ et al., 2008; Bui MH et al., 2003; Sandlund J et al., 2007). Es war sehr viel weniger häufig in papillären Karzinomen und nicht in normalgesundem Nierengewebe, in chromophoben sowie ductalen Karzinomen exprimiert (Liao SY et al., 1997; Sandlund J et al., 2007; Ivanov S et al., 2001; Murakami Y et al., 1999). Weitere Marker bzw. Oberflächenantigene werden unten stehend beschrieben.

Nach Auffassung der vorliegenden Erfinder ergeben sich vor dem Hintergrund neuer Erkenntnisse zu Tumorstammzellen ganz neue klinische Implikationen, die in Kombination mit den konventionellen therapeutischen Strategien eine kurative Behandlung von malignen Erkrankungen als greifbare Option erscheinen lassen. Bei einem entschlossenen Vorgehen zur Weiterentwicklung des Tumorstammzellmodells und der sich daraus ergebenden therapeutischen Möglichkeiten könnte ein neues Fenster in der internistischen Onkologie aufgestoßen werden.

Im Zusammenhang mit der vorliegenden Erfindung werden im Folgenden ferner virale Vektorsysteme erläutert: Diese können beispielsweise für den horizontalen Gentransfer verwendet werden. Der Vorgang der Infektion mit einem (bevorzugt replikationsinkompetenten) viralen Vektor wird als Transduktion der Zielzelle bezeichnet. Durch die viralen Vektoren können Transgene als Reporter (Alam J & Cook JL et al., 1990) in die Wirtszelle eingebracht werden. Die Verwendung eines Fluoreszenzproteins wie z.B. EGFP (Prasher DC et al., 1992; Chalfie M et al., 1994) ermöglicht eine schnelle Bestimmung der Transduktionseffizienz sowie in weiterführenden Untersuchungen die Bestimmung einer exklusiven Subpopulation. Des Weiteren können auch Antibiotikaresistenzgene als Reporter fungieren, die eine spätere Selektion der transduzierten Zellen ermöglichen; alternativ können auch funktionelle Transgene eingebracht werden. Des Weiteren bieten virale Vektorsysteme eine gute Möglichkeit, um die Expression von Transgenen über lange Zeiträume zu studieren. Die Verwendung unterschiedlicher Vektoren für den horizontalen Gentransfer erlaubt zudem zu untersuchen, ob ein beobachteter Effekt wirklich rein durch das eingebrachte Agens hervorgerufen wurde oder ob es mögliche Interaktionen und Beeinflussungen durch den verwendeten Vektor selbst gibt. Dabei weisen Viren aus der Familie der Retroviridae die Eigenschaft auf, dass sie ihr Genom in das Genom der infizierten Zelle integrieren. Retroviren besitzen ein einzelsträngiges RNA-Genom in Positivstrangorientierung, dies umfasst bei den verschiedenen Retroviren 7 bis 12 kb. Das Nukleokapsid ist von einer Hüllmembran umgeben und die Viruspartikel weisen eine Größe von ungefähr 100 nm auf. Das Genom der Retroviridae besteht aus zwei identischen Kopien einzelsträngiger RNA (*"single stranded* RNA", ssRNA), diese wird in DNA durch die reverse Transkription umgeschrieben und in das Genom der Wirtszelle integriert (Provirus). Der Aufbau eines retroviralen Genoms besteht an dem 5'- und 3'- Ende aus den *long terminal repeat*-Sequenzen (LTR), welche Zugleich als Promotor und als Verstärkungselement (*enhancer*) für die Expression funktionieren, sowie an der Integration in das Genom der Wirtszelle beteiligt sind. Sie sind aus repetitiven Bereichen bestehend aus den Regionen U3, R und U5 (*redundant,* R; *unique,* U) aufgebaut. Bestandteile eines jeden retroviralen Genoms sind die Gene env, gag und pol (Levy JA et al., 1995). Diese Gene kodieren für die Hüllproteine (env), die Enzyme Integrase, Protease und reverse Transkriptase (pol) und die gruppenspezifischen Antigene der Matrix- und Kapsidproteine (gag). Des Weiteren befindet sich eine Verpackungssequenz (ψ) zwischen der 5'-LTR und dem Startcodon für gag-pol, die für die Verpackung der RNA in die viralen Partikel notwendig ist (Mann R et al., 1983). Die ersten retroviralen Vektorsysteme basierten auf dem Moloney murinen Leukämie Virus (MoMLV). Diese Viren können nur sich teilende Zellen transduzieren, da sie über keinen aktiven Transportmechanismus durch die Kemmembran der Zielzelle verfügen. Nur wenn die Kemmembran während der Zellteilung aufgelöst wird, kann eine Integration des Virusgenoms in das Genom der Wirtszelle erfolgen (Roe T et al., 1993). Zu der Familie der Retroviridae gehören ebenfalls die Lentiviren, wie z.B. HIV-1 und SIV. Lentiviren weisen ein komplexeres Genom auf, welches im Vergleich zu den Retroviren für sechs zusätzliche akzessorische Proteine kodiert (tat, rev, vpr, vpu, nef und vif). Diese Viren sind im Gegensatz zu den Retroviren fähig auch ruhende Zellen zu infizieren, da sie in der Lage sind die Kemmembran aktiv zu überwinden (Naldini L et al., 1996; Fouchier RA et al., 1999). Diese Eigenschaft bietet die Möglichkeit Transgene in Zellen neuronalen Ursprungs einzubringen, was eine Untersuchung von persistierenden MV-Infektionen in Neuronen ermöglicht.

Allgemein bleibt festzustellen, dass im Fachgebiet trotz der oben erläuterten Ansätze noch unzureichend befriedigende Möglichkeiten der Identifikation und Gewinnung von Tumorstammzellen - und als Folge dessen auch bei der Diagnose und Therapie von malignen Erkrankungen - bestehen. Außerdem besteht nach wie vor ein Bedarf an einfachen, ökonomischen, wenig zeitaufwändigen und allgemein anwendbaren Verfahren zur Identifikation, Detektion und Gewinnung von Tumorstammzellen. Der vorliegenden Erfindung liegt unter anderem die Aufgabe zugrunde, der beschriebenen Problematik entgegenzuwirken und solche Verfahren bereitzustellen. So haben die vorliegenden Erfinder Ihre Bemühungen vor allem auch auf die weitere Entwicklung geeigneter Strategien zur Identifikation und Fraktionierung valider Tumorstammzellpopulationen aus verschiedenen Tumorentitäten fokussiert.

Der hier beschriebenen Erfindung liegt auch das Ziel zugrunde, Verfahren zur Verfügung zu stellen, welche die spezifische Detektion (Nachweis) und Gewinnung von humanen Tumorstammzellen und damit schließlich eine effektive - wenn möglich kurative - Therapie maligner Erkrankungen ermöglichen. Eine weitere Zielsetzung dieser Erfindung wird durch die nachfolgende proliferative Expansion dieser Zellen in einer definierten Zellkultur beschrieben. Die Erfindung basiert unter anderem auf der Erkenntnis, dass sich mononukleäre Zellen aus einem Gewebelysat, über einen Dichtegradienten mit anschließender Zentrifugation, aufreinigen lassen. Dies wird im Falle der Gewinnung von Tumorstammzellen aus aufgearbeiteten Tumorexplantaten beispielsweise über die Präparation einer Einzelzellsuspension genutzt. Ein zentraler hervorzuhebender Aspekt der vorliegenden Erfindung ist die Identifikation von Tumorstammzellen, die nachfolgend immunologisch spezifisch an eine bestimmte Tumorentität adressiert werden können. Ein weiteres Ziel der vorliegenden Erfindung ist, ein valides Zelltestsystem für wirkstoff- und zelltherapiebasierte Applikationen bereitzustellen, welches zudem eine reproduzierbare Tumorstammzellpopulation beinhaltet.

Erfindungsgemäße Aufgaben werden mit den hierin beschriebenen und beanspruchten Gegenständen gelöst. Eine ausführliche Darstellung der Verfahrenstechnologie sowie von Vorteilen und Eigenschaften der Erfindung wird nachfolgend bereitgestellt.

### Kurze Beschreibung der Zeichnungen

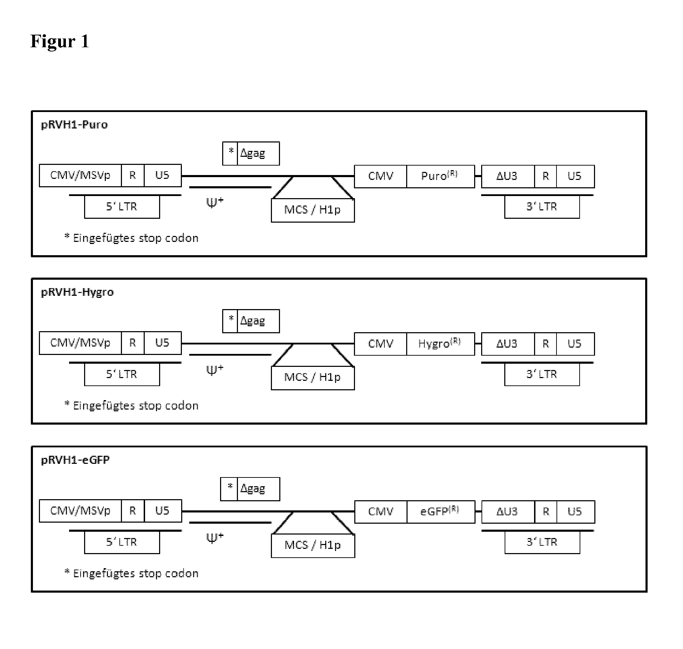

**Abbildung 1** ist eine schematische Darstellung der Plasmide zur Herstellung von viralen Vektoren basierend auf MoMLV.

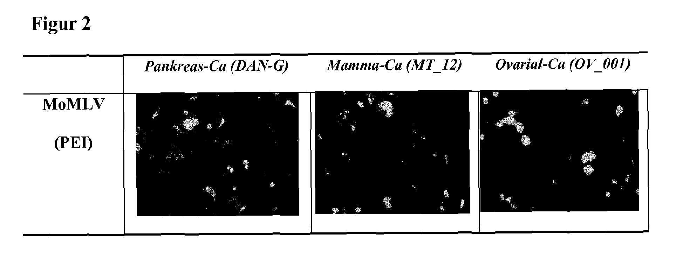

**Abbildung 2** ist eine repräsentative Darstellung der Transduktion einer Tumorzelllinie (DAN-G) und zweier primärer Tumoreinzelzellsuspensionen (MT_12 & OV_001) mit viralen Vektoren. Die viralen Vektoren basieren auf MoMLV. Die Herstellung der viralen Vektoren erfolgte über Transfektion mit PEI. Als Reportergen wird über die viralen Vektoren jeweils EGFP in die Tumorzellen eingebracht. Die Transduktion erfolgte mit 4 µg/ml Polybren ("Polybrene") und einem Zentrifugationsschritt für 90 min bei 200 g. Alle Aufnahmen sind bei lebenden Zellen und für einen Vergleich der Fluoreszenzintensität, unter den gleichen Bedingungen aufgenommen worden.

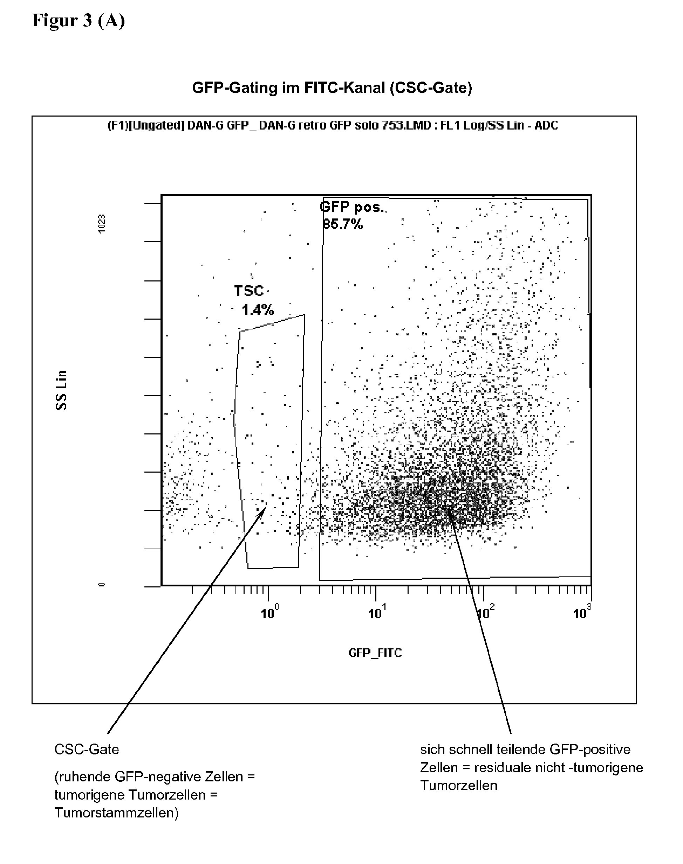

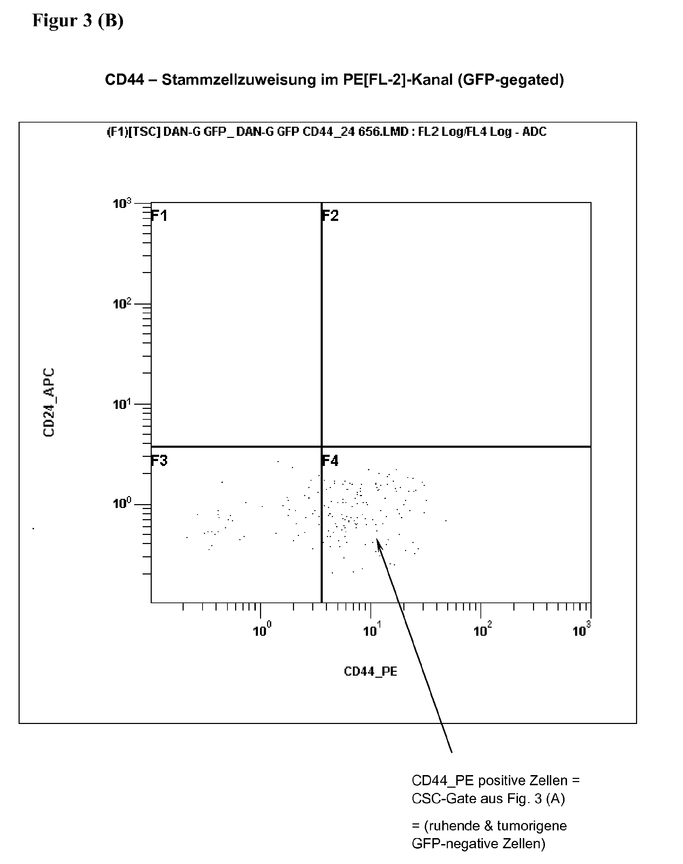

**Abbildung 3** **(A, B)** ist eine repräsentative durchflusszytometrische Darstellung der Tumorstammzellfraktion innerhalb einer MoMLV-basierten retroviral transduzierten Einzelzellsuspension einer humanen Pankreaskarzinomzelllinie. Panel A: Seitwärts-Scatter (Granulationsdichte der Zellen) versus Fluoreszenzkanal 1_GFP (FL_01, Absorption bei ca. 520nm), Panel B: Fluoreszenzkanal 4_CD24-APC (FL_4; 595-650 nm, APC = 633 nm) versus Fluoreszenzkanal 2_CD44-PE (FL_2; 488-546 nm, PE = 545 nm). In Panel A werden die ruhenden & GFP-negativen Zellen im TSC/CSC-Gate dargestellt. In Panel B wird dieses Gate dem Fluoreszenzkanal 2 mit einer CD44_PE-Konjugation zugewiesen

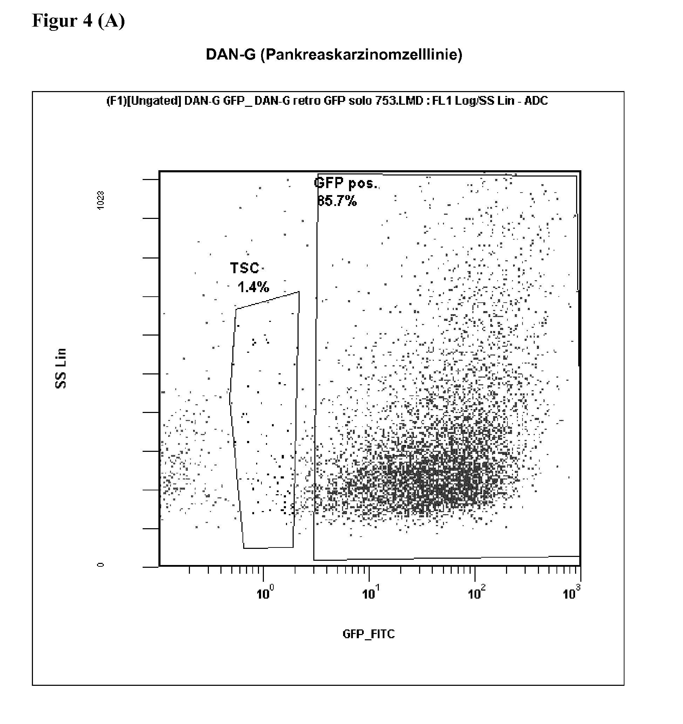

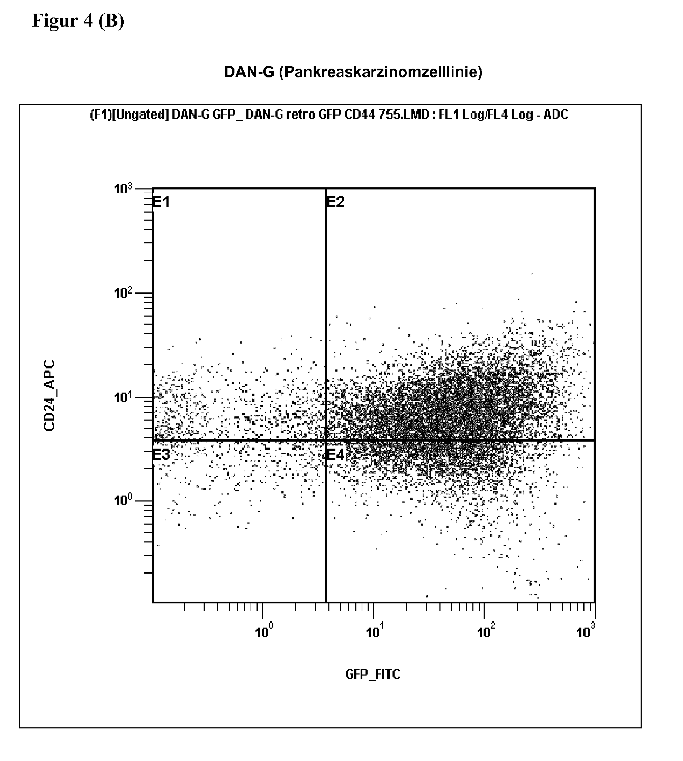

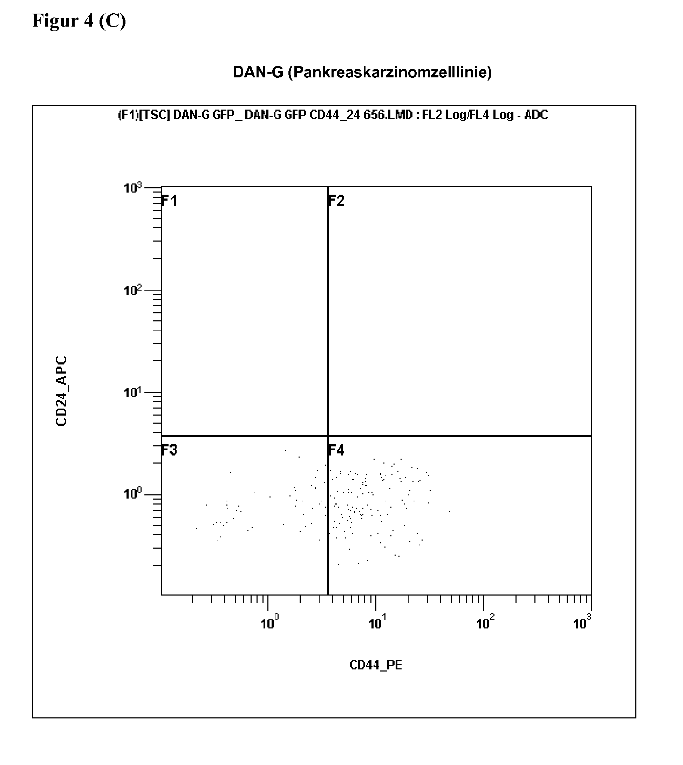

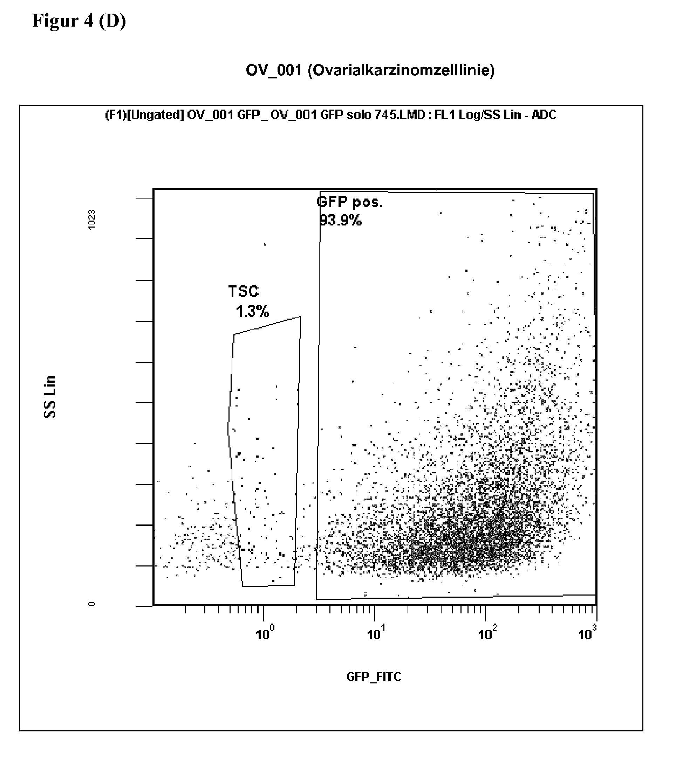

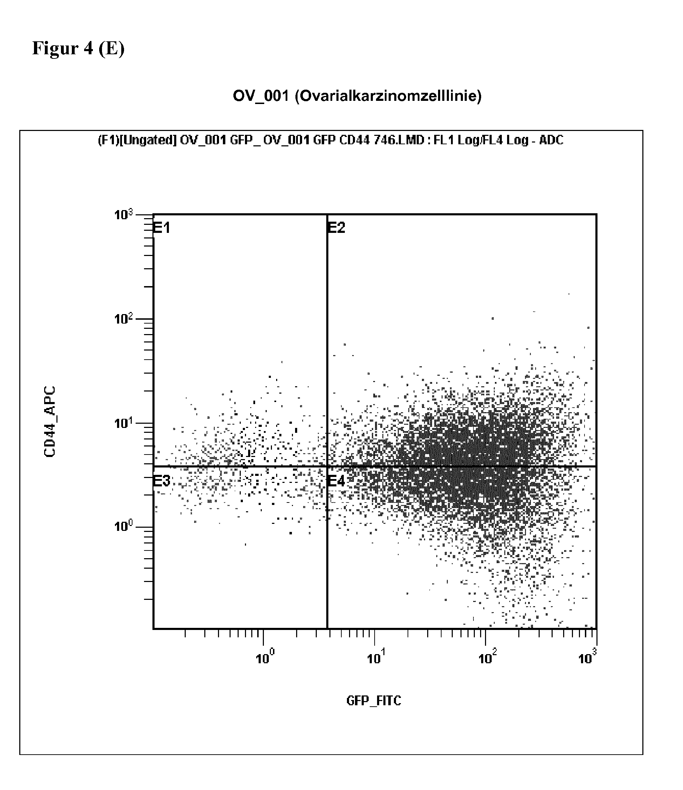

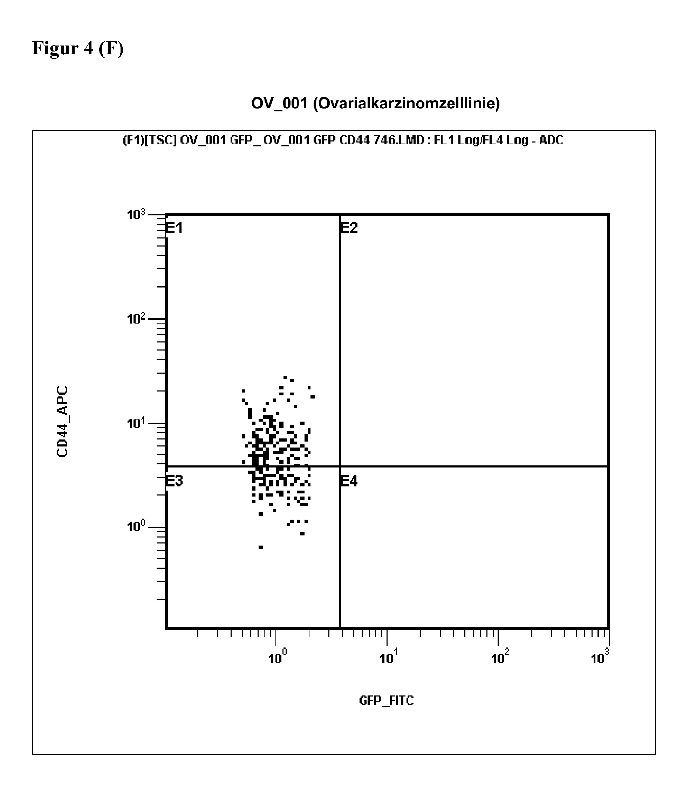

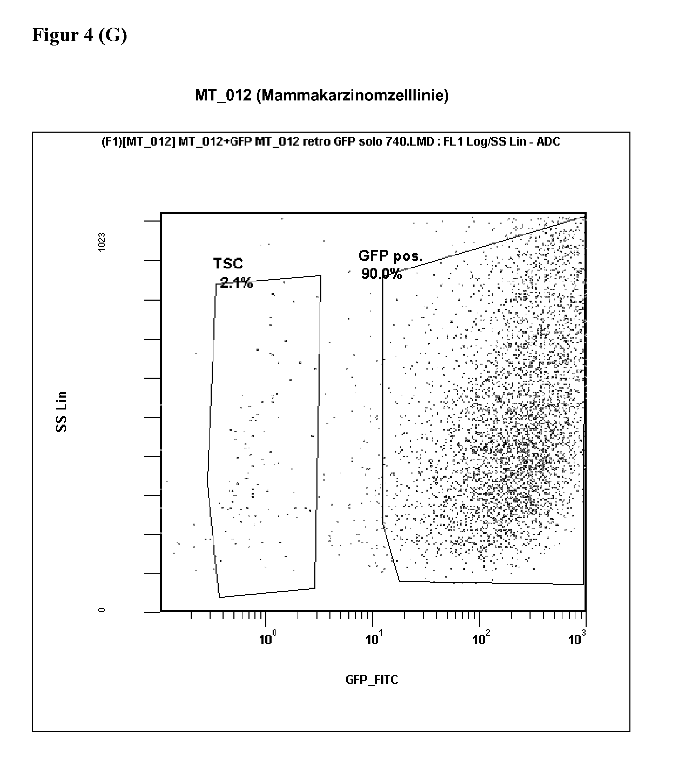

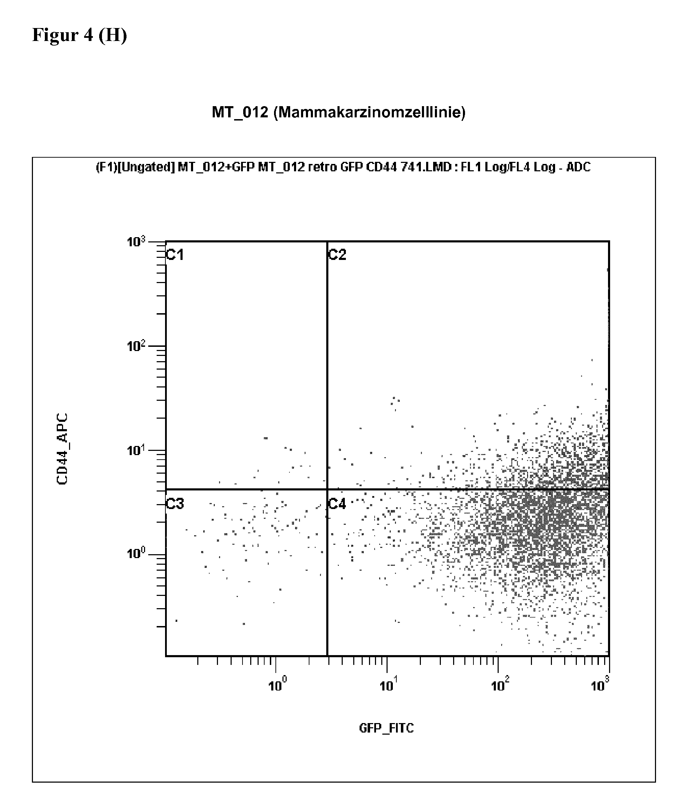

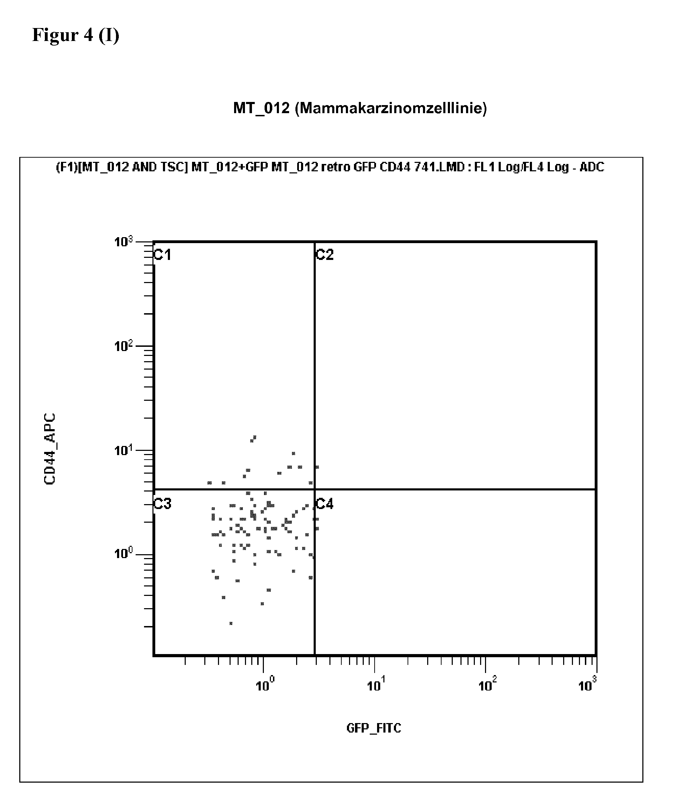

**Abbildung 4** **(A-I)** ist eine durchflusszytometrische Darstellung der Tumorstammzellfraktion innerhalb von MoMLV-basierten retroviral transduzierten Einzelzellsuspension humaner Karzinomzelllinien. *DAN-G:* Pankreaskarzinomzelllinie; Seitwärts-Scatter (Granulationsdichte der Zellen) versus Fluoreszenzkanal 1_GFP (FL_01, Absorption bei ca. 520nm) & Fluoreszenzkanal 4_CD24-APC (FL_4; 595-650nm, APC = 633nm) versus Fluoreszenzkanal 2_CD44-PE (FL_2; 488-546nm, PE=545nm). *OV_001:* Ovarialkarzinomzelllinie; Seitwärts-Scatter (Granulationsdichte der Zellen) versus Fluoreszenzkanal 1_GFP (FL_01, Absorption bei ca. 520nm) & Fluoreszenzkanal 1_GFP (FL_01; Absorption bei ca. 520nm) versus Fluoreszenzkanal 4_CD44-APC (FL_4; 595-650nm, APC = 633nm). *MT_012:* Mammakarzinom-Zelllinie; Seitwärts-Scatter (Granulationsdichte der Zellen) versus Fluoreszenzkanal 1_GFP (FL_01, Absorption bei ca. 520nm) & Fluoreszenzkanal L_GFP (FL_01; Absorption bei ca. 520nm) versus Fluoreszenzkanal 4_CD44-APC (FL_4, 595-650nm, APC = 633nm)

### Zusammenfassung der Erfindung

In einem ersten Aspekt betrifft die Erfindung ein Verfahren zur Detektion und/oder Identifikation von Tumorstammzellen in einer Tumorprobe, umfassend Auswählen von Tumorstammzellen basierend auf ihrem Zellteilungsverhalten, dadurch gekennzeichnet, dass Tumorzellen in der Tumorprobe, die keine Tumorstammzellen sind und ein im Vergleich zu Tumorstammzellen schnelleres Zellteilungsverhalten aufweisen, einer Markierung unterzogen werden.

In einem zweiten Aspekt betrifft die Erfindung ein Verfahren zur Gewinnung von Tumorstammzellen aus einer Tumorprobe, umfassend Auswählen von Tumorstammzellen basierend auf ihrem Zellteilungsverhalten, dadurch gekennzeichnet, dass Tumorzellen in der Tumorprobe, die keine Tumorstammzellen sind und ein im Vergleich zu Tumorstammzellen schnelleres Zellteilungsverhalten aufweisen, einer Markierung unterzogen werden.

In einem dritten Aspekt betrifft die Erfindung Tumorstammzellen, identifiziert nach einem Verfahren des ersten Aspekts, und erhältlich nach einem Verfahren des zweiten Aspekts, zur Verwendung in der Medizin.

In einem vierten Aspekt betrifft die Erfindung die Verwendung eines Mittels, geeignet zum Auswählen von Tumorstammzellen basierend auf ihrem Zellteilungsverhalten, i) bei der Detektion und/oder Identifikation von Tumorstammzellen in einer Tumorprobe und/oder ii) bei der Gewinnung von Tumorstammzellen aus einer Tumorprobe, dadurch gekennzeichnet, dass das Mittel die Markierung Tumorzellen in der Tumorprobe, die keine Tumorstammzellen sind und ein im Vergleich zu Tumorstammzellen schnelleres Zellteilungsverhalten aufweisen, ermöglicht.

Die Erfindung betrifft insbesondere die in den Ansprüchen definierten Gegenstände. Weitere Aspekte der Erfindung ergeben sich aus den nachfolgenden Ausführungen.

### Beschreibung der bevorzugten Ausführungsformen

Erfindungsgemäße Aufgaben werden beispielsweise durch eine hierin im Detail beschriebene spezifische Transduktions- und Immunfluoreszenz-basierte Sortierungsstrategie transformierter Zellen des Gesamtmalignoms - z.B. unter Einsatz replikationsdefizienter retroviraler Vektoren mit Markierung, beispielsweise GFP-Insertion - gelöst. Eine konkrete Strategie der vorliegenden Erfindung transduziert beispielsweise Gesamttumorzelllinien mittels eines GFP-inserierten retroviralen Vektorsystems, welches nur die sich in Teilung befindlichen Zellen infiziert. Schließlich kann eine spezifische Gating-Strategie der GFP-negativen Zellen verwendet werden, um alle ruhenden Zellen zu identifizieren und bevorzugt nach deren Zuweisung zu spezifischen Stammzellantigenen - als Tumorzellen mit Stammzelleigenschaften - zu fraktionieren und zu gewinnen.

Die vorliegende Erfindung verwendet im Wesentlichen eine Negativselektion ruhender Tumorzellen mit Stammzelleigenschaften. Zu diesem Zweck wird beispielsweise die gesamte Tumorprobe, beispielsweise in Form einer Einzelzellsuspension, mit einem retroviralen Vektor - der bevorzugt eine GFP-Insertion enthält - transduziert.

Wie bereits dargelegt, betrifft die Erfindung in einem ersten Aspekt Verfahren zur Detektion und/oder Identifikation von Tumorstammzellen in einer Tumorprobe, umfassend Auswählen von Tumorstammzellen basierend auf ihrem Zellteilungsverhalten, dadurch gekennzeichnet, dass Tumorzellen in der Tumorprobe, die keine Tumorstammzellen sind und ein im Vergleich zu Tumorstammzellen schnelleres Zellteilungsverhalten aufweisen, einer Markierung unterzogen werden.

Somit betrifft die Erfindung in diesem Aspekt unter anderem ein derart gekennzeichnetes Verfahren zur Detektion von Tumorstammzellen in einer Tumorprobe. Daneben betrifft die Erfindung in diesem Aspekt unter anderem ein derart gekennzeichnetes Verfahren zur Identifikation von Tumorstammzellen in einer Tumorprobe.

Wie hierin verwendet bezieht sich der Ausdruck "Detektion" bevorzugt auf den Nachweis von Tumorstammzellen, bevorzugt von bereits bekannten Tumorstammzellen, in einer Tumorprobe. Wie hierin verwendet umfasst der Ausdruck "Identifikation" bevorzugt den Nachweis von Tumorstammzellen, wie oben beschrieben. Darüber hinaus bezieht sich dieser Ausdruck ebenfalls auf den Nachweis zuvor unbekannter Tumorstammzellen in einer Tumorprobe. Dieser wird ebenfalls durch die hierin beschriebenen Vorgehensweisen ermöglicht und kann wiederum den Ausgangspunkt für eine nachfolgende "Detektion" dieser neu identifizierten Tumorstammzellen, beispielsweise in einer weiteren Tumorprobe, verwendet werden.

In bevorzugten Ausführungsformen ist das Verfahren nach dem ersten Aspekt ein *Ex-vivo-*Verfahren. In weiteren bevorzugten Ausführungsformen ist das Verfahren nach dem ersten Aspekt ein *In-vitro-*Verfahren. In gewissen Ausführungsformen umfasst das Verfahren nach dem ersten Aspekt den Schritt des Vorbereitens (hierin äquivalent "Bereitstellens") einer Tumorprobe. Das Vorbereiten (bzw. Erhalten) von Tumorproben ist keinen besonderen Beschränkungen unterworfen und ist dem Fachmann auf dem Gebiet hinlänglich bekannt. Bevorzugt umfasst das Verfahren des ersten Aspekts, insbesondere der Schritt Vorbereitens von Tumorproben, kein Verfahren zur chirurgischen oder therapeutischen Behandlung des menschlichen oder tierischen Körpers.

In einem zweiten Aspekt betrifft die Erfindung ein Verfahren zur Gewinnung von Tumorstammzellen aus einer Tumorprobe, umfassend Auswählen von Tumorstammzellen basierend auf ihrem Zellteilungsverhalten, dadurch gekennzeichnet, dass Tumorzellen in der Tumorprobe, die keine Tumorstammzellen sind und ein im Vergleich zu Tumorstammzellen schnelleres Zellteilungsverhalten aufweisen, einer Markierung unterzogen werden.

In bevorzugten Ausfiihrungsformen ist das Verfahren nach dem zweiten Aspekt ein *Ex-vivo-*Verfahren. In weiteren bevorzugten Ausfiihrungsformen ist das Verfahren nach dem zweiten Aspekt ein *In-vitro-*Verfahren*.* In gewissen Ausführungsformen umfasst das Verfahren nach dem zweiten Aspekt den Schritt des Vorbereitens einer Tumorprobe. Das Vorbereiten (bzw. Erhalten) von Tumorproben ist keinen besonderen Beschränkungen unterworfen und ist dem dem Fachmann auf dem Gebiet hinlänglich bekannt. Bevorzugt umfasst das Verfahren des zweiten Aspekts, insbesondere der Schritt Vorbereitens von Tumorproben, kein Verfahren zur chirurgischen oder therapeutischen Behandlung des menschlichen oder tierischen Körpers.

In bevorzugten Ausführungsformen umfasst das Verfahren nach dem zweiten Aspekt ein Verfahren nach dem ersten Aspekt der Erfindung und einen zusätzlichen Schritt des Gewinnens der besagten Tumorstammzellen. Ein solcher Schritt kann einen Einzelschritt oder ein oder mehrere Teilschritte umfassen. In gewissen Ausfiihrungsformen umfasst dieser Schritt das Abtrennen von Tumorzellen, die keine bzw. vorwiegend keine Tumorstammzellen sind. In gewissen Ausführungsformen umfasst dieser Schritt das Abtrennen von erfindungsgemäß markierten Zellen. In gewissen Ausführungsformen umfasst dieser Schritt das Abtrennen von Tumorstammzellen, die Gegenstand des erfindungsgemäßen Verfahrens sind aus der Probe. Somit umfasst erfindungsgemäßes "Gewinnen" bevorzugt die Auftrennung von in der Tumorprobe enthaltenen markierten und nicht markierten Zellen.

Wie hierin verwendet umfasst der Ausdruck "Gewinnung" bzw. "Gewinnen" das Erhalten von Tumorstammzellen in reiner Form, insbesondere unter Abtrennung weiterer in der Tumorprobe vorhandener Tumorzellen. Mit "in reiner Form" ist bevorzugt eine Reinheit von mindestens 50%, bevorzugt mindestens 60%, insbesondere mindestens 65%, 70%, oder 75%, insbesondere mindestens 80, 85%, oder 90%, insbesondere mindestens 95%, 97%, oder 99%, gemeint, wobei der Prozentsatz an Reinheit sich auf den Anteil von Tumorstammzellen an den nach Durchführung des Verfahrens in der Probe befindlichen Zellen bezieht. Insofern ist es gemäß der Erfindung keinesfalls erforderlich, dass die detektierten, identifizierten und/oder gewonnenen Tumorstammzellen absolut rein sind.

Die genannten Ausdrücke "Gewinnung" bzw. "Gewinnen" umfassen erfindungsgemäß ebenfalls die Fraktionierung der Tumorstammzellen. Die genannten Ausdrücke "Gewinnung" bzw. "Gewinnen" umfassen erfindungsgemäß ebenfalls die Abtrennung von Tumorstammzellen von weiteren Zellen, bevorzugt zur Erlangung einer oben beschriebenen Reinheit.

Die genannten Ausdrücke "Gewinnung" bzw. "Gewinnen" umfassen ebenfalls eine Anreicherung von Tumorstammzellen bevorzugt um einen Faktor von 2, stärker bevorzugt mindestens 4, wie z.B. von bis zu 5, 6, 7, 8, 9 oder 10, wie z.B. von bis zu 20, 30, 40 oder 50, bevorzugter von bis zu 100, 200, 500, noch bevorzugter von bis zu 1000, wobei bevorzugt auf einen Vergleich des ursprünglichen und erzielten Anteils von Tumorstammzellen an allen in der Tumorprobe enthaltenen Tumorzellen abgestellt wird. Die Ausdrücke "Gewinnung" bzw. "Gewinnen" umfassen daher erfindungsgemäß grundsätzlich ebenfalls die Anreicherung von Tumorstammzellen in einer Tumorprobe. Demgemäß betrifft der zweite Aspekt der vorliegenden Erfindung gleichermaßen ein Verfahren zur Anreicherung von Tumorstammzellen in einer Tumorprobe, umfassend Auswählen von Tumorstammzellen basierend auf ihrem Zellteilungsverhalten, dadurch gekennzeichnet, dass Tumorzellen in der Tumorprobe, die keine Tumorstammzellen sind und ein im Vergleich zu Tumorstammzellen schnelleres Zellteilungsverhalten aufweisen, einer Markierung unterzogen werden. Bevorzugte Ausführungsformen dieses Verfahrens entsprechen denen, die im Zusammenhang mit dem zweiten Aspekt hierin beschrieben sind.

Grundsätzlich wird der Fachmann erkennen, dass erfindungsgemäße Verfahren auch beinhalten können, dass zusammen mit Nicht-Tumorstammzellen auch Tumorstammzellen abgetrennt werden aber auch auf diese Weise eine Detektion, Identifikation und/oder Gewinnung der verbleibenden Tumorstammzellen erfolgen kann. Insofern ist gemäß der Erfindung nicht ausgeschlossen, dass auch Tumorstammzellen in einem gewissen Ausmaß der erfindungsgemäßen Markierung unterzogen werden. Wie hierin dargelegt ist dies den erfindungsgemäßen Verfahren nicht abträglich.

Die nachfolgende Offenbarung von bevorzugten Ausfiihrungsformen betrifft (auch wenn dies nicht ausdrücklich erwähnt ist) gleichermaßen zumindest die Verfahren des ersten und zweiten Aspekts, wobei die vorgenommenen Definitionen und Begriffserläuterung für alle erfindungsgemäßen Aspekte vorgesehen sind:
Erfindungsgemäß - und generell hierin - kann ein "Schritt" einen Einzelschritt darstellen oder aus (zwei oder mehreren) Teilschritten bestehen.

Wie u.a. an anderer Stelle hierin beschrieben, sind "Tumorstammzellen" (hierin beispielsweise auch als CSC, tumorigene Tumorzellen oder Tumor-initiierende Zellen bezeichnet) dem Fachmann bekannt. Wie hierin verwendet beinhaltet der Ausdruck "Tumorstammzellen" auch sogenannte Tumorzellen mit Stammzelleigenschaften.

Die genannten "Tumorzellen, die (keine Tumorstammzellen sind und) ein im Vergleich zu Tumorstammzellen schnelleres Zellteilungsverhalten aufweisen" sind dem Fachmann geläufig. Wie hierin verwendet bezieht sich der Ausdruck "Tumorzellen, die (keine Tumorstammzellen sind und) ein im Vergleich zu Tumorstammzellen schnelleres Zellteilungsverhalten aufweisen" bevorzugt auf die Tumorzellen, die den Großteil eines Tumors bzw. einer Tumorprobe darstellen. Diese sind durch relativ rasches Zellteilungsverhalten gekennzeichnet. Sie sind dem Fachmann gut bekannt und stellen beispielsweise das Ziel chemotherapeutischer Krebstherapieansätze dar.

Wie hierin verwendet ist eine "Tumorprobe" (hierin auch als "Probe" bezeichnet) keinen Beschränkungen unterworfen. Dem Fachmann ist die Gewinnung von Tumorproben hinlänglich bekannt. Bevorzugte Ausführungsformen für Tumorproben ergeben sich u.a. aus der nachfolgenden Offenbarung einschließlich der Beispiele. Bevorzugt ist vorgesehen, dass als eine Tumorprobe eine aus einer ursprünglichen Tumorprobe *ex vivo* hergestellte Einzelzellsuspension eingesetzt wird. Gemäß bevorzugten Ausführungsformen ist die Tumorprobe ein Gewebeexplantat (hierin auch "Tumorexplantat"), bevorzugt eine aus einem Gewebeexplantat hergestellte Einzelzellsuspension. Auch die hierzu gehörigen Verfahren liegen innerhalb des Könnens eines Fachmanns auf dem Gebiet.

In bevorzugten Ausfiihrungsformen stellt das Auswählen in einem erfindungsgemäßen Verfahren eine Negativauswahl dar. Wie hierin verwendet bezieht sich der Ausdruck "Negativauswahl" auf eine indirekte Auswahl. Dabei wird bevorzugt aufgrund einer (bevorzugt im Laufe des Verfahrens verursachten) Eigenschaft ausgewählt, die die zur Frage stehenden Zellen nicht aufweisen. In einem konkreten, nicht limitierenden Beispiel, ist diese Eigenschaft die mangelnde Aufnahmemöglichkeit und/oder das mangelnde Vorhandensein einer Markierung, wie hierin beschrieben.

In bevorzugten Ausführungsformen des Verfahrens nach dem ersten oder zweiten Aspekt werden die genannten Tumorzellen in der Tumorprobe die keine Tumorstammzellen sind und ein im Vergleich zu Tumorstammzellen schnelleres Zellteilungsverhalten aufweisen einer selektiven Markierung unterzogen.

Wie hierin verwendet bedeutet der Ausdruck "selektive Markierung" insbesondere, dass die Markierung vorwiegend von Tumorzellen in der Tumorprobe, die keine Tumorstammzellen sind, aufgenommen wird. Bevorzugt wird die Markierung von Tumorstammzellen im Vergleich zu den genannten Tumorzellen mit schnellerem Zellteilungsverhalten um einen Faktor von 2, stärker bevorzugt mindestens 4, wie z.B. von bis zu 5, 6, 7, 8, 9 oder 10, wie z.B. von bis zu 20, 30, 40 oder 50, bevorzugter von bis zu 100, 200, 500, noch bevorzugter von bis zu 1000 oder mehr, besser bzw. schneller aufgenommen. Bevorzugt wird die Markierung von einer geringeren Zahl von Tumorstammzellen im Vergleich zu den genannten Tumorzellen mit schnellerem Zellteilungsverhalten aufgenommen, wobei sich die bevorzugten Unterschiede der Anzahlen der Zellen aus den oben genannten Faktoren ergeben. Allgemein werden Tumorstammzellen erfindungsgemäß vorzugsweise keiner Markierung, bzw. einer geringeren Markierung (bzw. Markierung in geringerem Umfang), insbesondere einer gemäß der im Zusammenhang mit der "Gewinnung" als bevorzugt beschriebenen Faktoren geringeren Markierung, unterzogen als die besagten Tumorzellen, die der Markierung unterzogen werden.

Wie hierin verwendet bedeutet der Ausdruck "ein im Vergleich zu Tumorstammzellen schnelleres Zellteilungsverhalten" insbesondere, dass die betreffenden Zellen sich in der Tumorprobe unter den vorliegenden Bedingungen schneller teilen als die Tumorstammzellen. Dabei ist u.a. beabsichtigt, dass sich der Ausdruck "schnelleres Zellteilungsverhalten" bevorzugt auf ein Zellteilungsverhalten bezieht, dass um einen Faktor von 2, stärker bevorzugt mindestens 4, wie z.B. von bis zu 5, 6, 7, 8, 9 oder 10, wie z.B. von bis zu 20, 30, 40 oder 50, bevorzugter von bis zu 100, 200, 500, noch bevorzugter von bis zu 1000 oder mehr schneller ist. Die konkrete Bestimmung von Zellteilungsverhalten und Zellteilungsgeschwindigkeiten ist dem Fachmann gut bekannt.

In weiteren bevorzugten Ausführungsformen des Verfahrens nach dem ersten oder zweiten Aspekt wird die Markierung über einen Vektor, bevorzugt viralen Vektor, stärker bevorzugt retroviralen Vektor, in die genannten Tumorzellen in der Tumorprobe, die ein im Vergleich zu Tumorstammzellen schnelleres Zellteilungsverhalten aufweisen, eingeführt, insbesondere wobei der Vektor nur von sich teilenden Zellen aufgenommen wird, im Besonderen wobei der Vektor replikationsinkompetent ist, bevorzugt wobei der Vektor auf dem Retrovirus MoMLV basiert, insbesondere wobei der Vektor unter Verwendung von Phoenix-Zellen hergestellt wurde.

Somit wird erfindungsgemäß bevorzugt ein retroviraler Vektor bei der Auswahl von Tumorstammzellen verwendet. Dieser Vektor ermöglicht vorzugsweise die selektive Markierung der genannten Tumorzellen, die keine Tumorstammzellen darstellen.

Allgemein wird in Verfahren des ersten oder zweiten Aspekts der Vektor (bzw. die Markierung) bevorzugt "vorwiegend" (d.h. insbesondere mittels der hierin definierten Faktoren) bzw. stärker bevorzugt "im Wesentlichen" ausschließlich von sich teilenden Zellen aufgenommen.

Gemäß der vorliegenden Erfindung erfolgt das genannte "Einführen" (insbesondere das Einführen der Markierung) vorzugsweise mittels Transduktion. Gemäß der vorliegenden Erfindung bedeutet die genannte "Aufnahme" vorzugsweise Aufnahme, insbesondere Aufnahme der Markierung, in den Zellkern. Somit findet in bevorzugten Ausfiihrungsformen der erfindungsgemäßen Verfahren ein Transport der Markierung in den Zellkern der Zielzelle (d.h. bevorzugt die hierin definierten Tumorzellen, die keine Tumorstammzellen sind) statt.

Die Ausdrücke "Vektor" und "viraler Vektor", wie hierin verwendet, beziehen sich somit bevorzugt auf Viruspartikel, vorzugsweise retrovirale Vektorsysteme bzw. Viruspartikel.

In bevorzugten Ausfiihrungsformen ist der Vektor replikationsinkompetent. In bevorzugten Ausführungsformen basiert der Vektor auf dem Retrovirus MoMLV ("Moloney Murine Leukemia Virus"). In bevorzugten Ausführungsformen wird der Vektor unter Verwendung von Phoenix-Zellen hergestellt.

In weiteren bevorzugten Ausführungsformen des Verfahrens nach dem ersten oder zweiten Aspekt ist die Markierung eine Fluoreszenzmarkierung, bevorzugt eine GFP-Markierung, insbesondere wobei die Fluoreszenzmarkierung über einen Vektor in die genannten Tumorzellen in der Tumorprobe, die ein im Vergleich zu Tumorstammzellen schnelleres Zellteilungsverhalten aufweisen, eingeführt wird, wobei der Vektor bevorzugt wie oben beschrieben definiert ist, im Besonderen wobei das Einführen mittels Transduktion erfolgt, insbesondere wobei der Vektor aus in Phönixzellen produzierten Viruspartikeln besteht, mit denen die Tumorzellen infiziert werden.

Somit wird in Verfahren des ersten oder zweiten Aspekts bevorzugt die Fluoreszenzmarkierung über einen Vektor in die genannten Tumorzellen in der Tumorprobe, die ein im Vergleich zu Tumorstammzellen schnelleres Zellteilungsverhalten aufweisen, eingeführt. In bevorzugten Ausfiihrungsformen ist der Vektor wie oben beschrieben ausgestaltet.

Somit erfolgt in Verfahren des ersten oder zweiten Aspekts das Einführen der Markierung bzw. des Vektors bevorzugt mittels Transduktion. Transduktionsverfahren sind dem Fachmann hinlänglich bekannt. In bevorzugten Ausfiihrungsformen besteht der Vektor aus Viruspartikeln, insbesondere aus in Phönixzellen produzierten Viruspartikeln, mit denen die Tumorzellen infiziert werden.

Somit ist die Fluoreszenzmarkierung gemäß dem ersten oder zweiten Aspekt bevorzugt ein GFP ("green fluorescent protein"), wie z.B. EGFP ("enhanced green fluorescent protein"). Alternativ ist die Fluoreszenzmarkierung bevorzugt ein YFP ("yellow fluorescent protein") Alternativ ist die Fluoreszenzmarkierung bevorzugt ein RFP ("red fluorescent protein").

In weiteren bevorzugten Ausführungsformen des Verfahrens nach dem ersten oder zweiten Aspekt, umfasst die Detektion und/oder Identifikation bzw. die Gewinnung weiterhin Bestimmen von mindestens einem Marker, bevorzugt mindestens einem Oberflächenmarker, auf den Tumorstammzellen.

Generell umfasst die vorliegende Erfindung in bevorzugten Ausführungsformen des ersten Aspekts Verfahren, in denen die Detektion, und/oder Identifikation von Tumorstammzellen (insbesondere von speziellen Tumorstammzellen) auf Basis des Bestimmens von Markern erfolgt. In weiteren Ausführungsformen der Verfahren des ersten Aspekts wird das allgemeine Vorhandensein von Tumorstammzellen detektiert. Somit ist nicht erforderlich, aber bevorzugt, dass die detektierten bzw. identifizierten Tumorstammzellen weiter charakterisiert werden (insbesondere im Detail charakterisiert werden) und/oder einer bestimmten Tumorart zugeordnet werden. Bevorzugt umfassen somit insbesondere die Verfahren des ersten Aspekts das Bestimmen mindestens eines Markers. Somit umfassen erfindungsgemäße Verfahren bevorzugt die Auswahl von Tumorstammzellen auf Basis des Vorhandenseins mindestens eines Markers.

Alternativ oder zusätzlich wird eine Gruppe von Markern (insbesondere Oberflächenmarkern), bevorzugt umfassend Stammzellmarker, Adhäsionsmarker, und/oder Tumormarker bestimmt. Besonders bevorzugt sind Stammzellmarker. Besonders bevorzugt sind Tumormarker.

Die vorliegende Erfindung betrifft somit in bevorzugten Ausführungsformen Verfahren, in denen oben bezeichneten (Gruppen von) Marker(n) bestimmt werden. In bevorzugten Ausfiihrungsformen werden mindestens zwei Marker, bevorzugt mindestens zwei Stammzellmarker bestimmt. In manchen Ausführungsformen werden 3 bis 10 Marker bestimmt. In manchen Ausführungsformen werden mindestens 3 Marker bestimmt. In manchen Ausfiihrungsformen werden mindestens 4 Marker bestimmt. In manchen Ausfiihrungsformen werden mindestens 5 Marker bestimmt. In manchen Ausfiihrungsformen werden mindestens 6 Marker bestimmt. In manchen Ausführungsformen werden 7 Marker bestimmt. Eine Gruppe von Markern kann dabei aus beliebigen der beschriebenen Markertypen zusammengesetzt sein. Das Bestimmen der Marker kann erfindungsgemäß generell auch sequentiell durchgeführt werden.

Alternativ oder zusätzlich ist der Marker, vorzugsweise der Oberflächenmarker, ausgewählt aus der Gruppe, bestehend aus CA_09, CD10, CD11, CD24, CD34, CD38, CD44, CD45, CD73, CD133, CD326, CXCR4 und EpCAM.

Somit wird in Verfahren des ersten oder zweiten Aspekts bevorzugt eine Gruppe von Oberflächenmarkern, umfassend Stammzellmarker, Adhäsionsmarker, und/oder Tumormarker bestimmt. Bevorzugt umfasst die Gruppe von Oberflächenmarkern Stammzellmarker. Bevorzugt umfasst die Gruppe von Oberflächenmarkern Tumormarker. Bevorzugt umfasst die Gruppe von Oberflächenmarkern Stammzellmarker und Tumormarker.

Somit wird in Verfahren des ersten oder zweiten Aspekts der Marker, vorzugsweise der Oberflächenmarker, bevorzugt ausgewählt aus der Gruppe, bestehend aus CA_09, CD10, CD11, CD24, CD34, CD38, CD44, CD45, CD73, CD133, CD326, CXCR4 und EpCAM. Unter diesen Markern sind CD34, CD38, CD44 und das CD133 Beispiele für bevorzugte Stammzellmarker. Unter diesen Markern sind CD24, CD34, CD38, CD45 und CD326 Beispiele für bevorzugte Adhäsionsmoleküle bzw. Adhäsionsmarker. Unter diesen Markern sind CA09, CD10, CD11, CXCR4 und CD326 Beispiele für bevorzugte Tumormarker.

Nachfolgend werden im Zusammenhang mit der Erfindung beispielhafte Marker bzw. Oberflächenmarker weiter erläutert. Der Fachmann wird erkennen, dass diese Auflistung rein exemplarisch ist und innerhalb des Umfangs der Erfindung jeglicher Marker und jegliche Kombination von Markern verwendet werden kann:
CD44 ist ein Rezeptor für Hyaluronsäure und der auch mit anderen Liganden wie Osteopontin, Kollagen und Matrix-Metalloproteinasen (MMPs) interagieren kann. Die CD44-Glykoform wurde ursprünglich auf menschlichen hämatopoetischen Stammzellen und leukämischen Blasten entdeckt und anschließend auf Krebszellen identifiziert. CD44 fungiert als "Homing-Rezeptor" für Stammzellen im Knochenmark. Er übernimmt auch die Regie bei der Migration von humanen hämatopoetischen- und mesenchymalen Stammzellen des Knochenmarks. CD44 Gen-Transkription ist mindestens zum Teil durch beta-Catenin und Wnt-Signalweg (auch Tumor-Entwicklung verbunden) aktiviert. CD133, ursprünglich als AC133 bekannt. CD133 ist ein Glykoprotein auch in Menschen und Nagetieren als Prominin 1 bekannt. CD133 ist in hämatopoetische Stammzellen, endotheliale Vorläuferzellen, Glioblastom, neuronalen und glialen Stammzellen verschiedenen pädiatrischen Hirntumoren sowie erwachsenen Nieren, Brustdrüsen, Luftröhre, Speicheldrüsen, Plazenta, Verdauungstrakt, Hoden exprimiert. Jüngste Studien bei Hirntumoren haben eine CD133-Zellpopulation identifiziert, die als sogenannte Tumorstammzellpopulation gelten kann. CD24 ist ein Glykoprotein auf der Oberfläche der meisten B-Lymphozyten und differenzierenden Neuroblasten exprimiert. Dieses Gen kodiert für ein Sialoglycoprotein, die auf reifen Granulozyten und in vielen B-Zellen exprimiert wird. Das codierte Protein wird über einen Glycosyl-Link auf der Zelloberfläche verankert. Bezüglich der Identifikation von CSC, kann über seine Expression die Präsenz von Zellen epidermalen Ursprungs ausgeschlossen werden. EpCAM ist ein pan-epitheliales Differenzierungsantigen, das auf fast allen Karzinomen exprimiert wird. Es ist eng mit dem Cadherin-Catenin Signalweg und damit dem grundlegenden Wnt-Signalweg verbunden. Es ist in undifferenzierten pluripotenten Stammzellen exprimiert. CA_09 ist ein Tumormarker des hellzelligen Nierenzellkarzinoms. Weitere Eigenschaften von CA_09, die gleichzeitig bevorzugte Merkmale gemäß der vorliegenden Erfindung darstellen, sind im Einleitungsteil hierin beschrieben. CXCR4 ist ein Tumormarker des metastasierenden Pankreaskarzinoms.

In beispielhaften bevorzugten Verfahren der vorliegenden Erfindung weist die Gruppe von Markern CD44+, CD24-, CXCR4- auf Tumorstammzellen aus Pankreaskarzinom (Primärtumor; die Metastase ist CXCR4-positiv) hin. In weiteren beispielhaften Verfahren der vorliegenden Erfindung weist die Gruppe von Markern CD44+, EpCAM+, CD24-, CA09- auf Tumorstammzellen aus Nierenzellkarzinom hin. In weiteren beispielhaften Verfahren der vorliegenden Erfindung weist die Gruppe von Markern CD44+, CD24- auf Tumorstammzellen aus Mamma-, Ovarial- bzw. Prostatakarzinom hin. Somit beinhalten bestimmte Ausführungsformen erfindungsgemäßer Verfahren das Bestimmen der oben beschriebenen Kombinationen / Gruppen von Markern. Der Fachmann wird ohne Weiteres erkennen, dass die Kombinationen von Markern keinen besonderen Beschränkungen unterworfen sind und welche weitere(n) Kombinationen für die jeweilige zur Frage stehende Anwendung insbesondere in Frage kommen. Weitere beispielhafte Kombinationen bzw. Gruppen von Markern ergeben sich aus der Offenbarung in den Beispielen.

In weiteren bevorzugten Ausführungsformen des Verfahrens nach dem ersten oder zweiten Aspekt wird der mindestens eine Oberflächenmarker oder die Gruppe von Oberflächenmarkern unter Verwendung von mindestens einem markierten Antikörper bestimmt, insbesondere wobei der mindestens eine markierte Antikörper eine Fluoreszenzmarkierung trägt, im Besonderen wobei für verschiedene Oberflächenmarker verschiedene Fluoreszenzmarkierungen, bevorzugt ausgewählt aus Flurescein, Phycoerythrin und Allophycocyanin, verwendet werden, bevorzugt wobei sich die Fluoreszenzmarkierung(en) von der im Zuge der (Negativ)auswahl von Tumorstammzellen definierten Markierung unterscheidet/n.

Das heißt, in erfindungsgemäßen Verfahren werden die Marker (z.B. die in der Gruppe von Markern enthaltenen Marker), insbesondere die Oberflächenmarker, bevorzugt unter Verwendung von mindestens einem Antikörper, bevorzugt markiertem Antikörper, insbesondere fluoreszenz-markiertem Antikörper bestimmt. Beispielhafte Fluoreszenzmarkierungen sind ausgewählt aus Flurescein, Phycoerythrin und Allophycocyanin. Bevorzugt unterscheidet/n sich die Fluoreszenzmarkierung(en) von der im Zuge der (Negativ)auswahl definierten Markierung. Eine geeignete Strategie zum Einsatz verschiedener Mittel zum vorteilhaftem Nachweis verschiedener Marker ist dem Fachmann ohne Weiteres zugänglich.

In weiteren bevorzugten Ausführungsformen umfassen Verfahren nach dem ersten oder zweiten Aspekt das Herstellen einer Einzelzellsuspension aus der Tumorprobe, insbesondere wobei das Auswählen der Tumorstammzellen aus einer Einzelzellsuspension erfolgt, im Besonderen wobei das Verfahren die Verwendung von Durchflusszytometrie, insbesondere von FACS-Analyse umfasst. Somit ist in bestimmten Ausführungsformen die in den erfindungsgemäßen Verfahren eingesetzte Tumorprobe eine aus einer ursprünglichen Tumorprobe (wie z.B. eine direkt aus einem Patienten entnommene ursprüngliche Tumorprobe) hergestellte, bevorzugt *ex vivo* hergestellte, Einzelzellsuspension.

Allgemein umfassen erfindungsgemäße Verfahren bevorzugt die Verwendung von Durchflusszytometrie, insbesondere von FACS-Analyse. Somit wird erfindungsgemäß bevorzugt Durchflusszytometrie, insbesondere FACS-Analyse, zur Detektion, Identifikation und/oder Gewinnung von Tumorstammzellen verwendet.

In weiteren bevorzugten Ausführungsformen umfassen Verfahren nach dem ersten oder zweiten Aspekt einen Kontrollschritt, um das Auswählen apoptotischer und/oder nekrotischer Zellen zu vermeiden. Gleichermaßen umfassen in weiteren bevorzugten Ausführungsformen Verfahren nach dem ersten oder zweiten Aspekt einen Schritt zum Abtrennen apoptotischer und/oder nekrotischer Zellen. Diese Schritte können aus einzelnen Schritten bestehen oder auch mehrere Schritte umfassen.

Solche Schritte sind dem Fachmann - insbesondere auch unter Berücksichtigung der vorliegenden Beschreibung gut bekannt. Beispielsweise kann der Ausschluss bzw. die Eliminierung von toten oder apoptotischen Zellen über Annexin V- und 7-AAD-Analyse erfolgen. Bevorzugt erfolgt sie vor einer solchen Zuweisung ebenso über eine Fluoreszenzzellsortierung.

Der Nachweis der Apoptose erfolgt beispielsweise mit Hilfe von Annexin-V-PE/7-AAD an unbehandelten und GFP-transduzierten Tumorzellen. Die Messung der Apoptose erfolgt beispielsweise an einem "FACScan"-Durchftusszytometer (FC500; Beckman Coulter) und wurde im Zusammenhang mit der vorliegenden Erfindung beispielsweise auf die in diesem Absatz beschriebene Art und Weise durchgeführt. Während der durchflusszytometrischen Messung werden beispielsweise 10 000 Zellen aufgenommen und die Fluoreszenz von Annexin V-PE (FL-2) und 7-AAD (FL-4) wird gemessen. Dies wird dann mit der GFP (FL-1)- Fluoreszenz der nicht-tumorigenen Tumorzellen ins Verhältnis gesetzt. Zur Auswertung werden die Zellen beispielsweise in einem Dotplot-Diagramm, in dem FL-4 gegen FL-2 aufgetragen wurde, dargestellt. In einem solchen Diagramm repräsentiert jeder abgebildete Punkt (Dot) eine Zelle der Probe. Zellen mit Annexin-V-/7-AAD- stellen die lebende Fraktion innerhalb der Probe dar. Die Zellen mit Annexin-V+/7-AAD- sind früh apoptotisch, da sie Phosphatidylserin externalisiert haben, an das Annexin-V bindet, und noch eine intakte Plasmamembran besitzen. Die Zellen, die Positivität für beide Fluoreszenzen - Annexin-V+/7AAD+ - zeigen, sind nekrotische Zellen und werden bei der späteren Separation ausgegrenzt. Da die Majorität der Tumorzellen durch ihre GFP-Transduktion im FL-1 grün fluoresziert, kann in vier verschiedenen Dotplots jeweils die Apoptose- und die Vitalitätsrate der Tumorstammzellen bestimmt werden. Weitere bevorzugte Ausführungsformen solcher (Kontroll)schritte beinhalten die Bestimmung des Granulationsgrades von Zellen im Seitwärts-Scatter, beispielsweise wie hierin unterhalb beschrieben.

Darüber hinaus könnten gleichsam postmitotische Bindegewebszellen, die auch als "ruhend" erscheinen können, sich aber dann nicht mit Stamm- oder Tumorzelleigenschaften darstellen lassen, in der Tumorprobe enthalten sein. In bevorzugten Ausführungsformen umfassen erfindungsgemäße Verfahren daher einen oder mehrere (Kontroll)schritt(e), um das Auswählen letzterer Zellen zu vermeiden bzw. diese abzutrennen. Solche Schritte sind dem Fachmann ebenfalls ohne weiteres geläufig. Bevorzugt umfasst/umfassen diese(r) Schritt(e) die Bestimmung mindestens eines Stammzellmarkers, insbesondere die Auswahl von Tumorstammzellen auf Basis des Vorhandenseins dieses Stammzellmarkers. Bevorzugt umfasst/umfassen diese(r) Schritt(e) die Bestimmung mindestens eines Tumormarkers, insbesondere die Auswahl von Tumorstammzellen auf Basis des Vorhandenseins dieses Tumormarkers.

Generell können hierin beschriebene (Kontroll)Schritt(e) auch indirekt und im Zusammenhang mit nachfolgenden Verfahrensschritten erfolgen. Beispielsweise können die Schritte dadurch erfolgen, dass Tumorstammzellen im Laufe des Verfahrens aktiv von weiteren Zellen in der Probe abgetrennt werden.

In bevorzugten Verfahren nach dem ersten und zweiten Aspekt sind die Tumorstammzellen ausgewählt aus der Gruppe, bestehend aus Tumorstammzellen aus Mammakarzinom, Ovarialkarzinom, Pankreaskarzinom, Prostatakarzinom, Urothelkarzinom und Nierenzellkarzinom. Diese Auflistung ist jedoch nicht als Beschränkung gedacht, da der Fachmann erkennen wird, dass die Erfindung auf im Wesentlichen alle Arten von Tumorstammzellen Anwendung findet. Besonders bevorzugt sind die Tumorstammzellen aus Mammakarzinom. Besonders bevorzugt sind die Tumorstammzellen aus Pankreaskarzinom. Besonders bevorzugt sind die Tumorstammzellen aus Ovarialkarzinom.

In bestimmten Ausführungsformen des ersten und zweiten Aspekts wird die detektierte, bestimmte bzw. gewonnene Tumorstammzellfraktion einer weiteren Validierung zugeführt. Nicht beschränkende Beispiele hierfür sind Anwachsen im (nicht-menschlichen) Versuchstier, im Vergleich zu den depletierten, sich schnell teilenden und nicht-tumorigenen Tumorzellen (diese dürfen nicht anwachsen). Alternativ bzw. zusätzlich kann eine weitere Charakterisierung, beispielsweise über RNA-Chip-Technologie (z.B. Top-Kandidaten für Überexpression) und/oder Western Blot (z.B. Expression von Genprodukten der Stammzell-Pathways) erfolgen.

In einem dritten Aspekt betrifft die Erfindung Tumorstammzellen, identifiziert nach einem Verfahren des ersten Aspekts, und erhältlich nach einem Verfahren des zweiten Aspekts, zur Verwendung in der Medizin. Bevorzugt verwenden mittels eines Verfahrens nach dem ersten Aspekt erstmals identifizierte Tumorstammzellen eingesetzt.

Bevorzugte Ausführungsformen des dritten Aspekts ergeben sich aus denen des ersten und/oder zweiten Aspekts. Insofern wird diesbezüglich auf die oben stehenden Ausführungen verwiesen. Weitere Ausführungsformen des dritten Aspekts ergeben sich aus den Gegenständen der unten offenbarten weiteren Aspekte der Erfindung sowie aus den weiteren hierin beschriebenen Verwendungsmöglichkeiten von Tumorstammzellen.

Beispielhafte Anwendungen in der Medizin beinhalten die Untersuchung der Tumorstammzellen im Hinblick auf Wirkstoffe gegen die zugehörigen Tumorerkrankungen, beispielsweise durch ein Screening von Wirkstoffkandidaten. Diese Wirkstoffkandidaten können bereits im Zusammenhang mit einer weiteren Tumorerkrankung bekannt sein oder nicht. Eine weitere Anwendung in der Medizin stellt die Diagnose von Tumorerkrankungen dar. Eine beispielhafte weitere Anwendung stellt die Vervielfältigung der gewonnenen Tumorstammzellen, beispielsweise unter Erhalt entsprechender Zellkulturen, dar.

In einem vierten Aspekt betrifft die Erfindung die Verwendung eines Mittels, geeignet zum Auswählen von Tumorstammzellen basierend auf ihrem Zellteilungsverhalten, i) bei der Detektion und/oder Identifikation von Tumorstammzellen in einer Tumorprobe und/oder ii) bei der Gewinnung von Tumorstammzellen aus einer Tumorprobe, dadurch gekennzeichnet, dass das Mittel die Markierung Tumorzellen in der Tumorprobe, die keine Tumorstammzellen sind und ein im Vergleich zu Tumorstammzellen schnelleres Zellteilungsverhalten aufweisen, ermöglicht.

Bevorzugt stellt das Auswählen der Tumorstammzellen des vierten Aspekts eine Negativauswahl dar, die bevorzugt wie anderswo hierin definiert ausgestaltet ist.

Bevorzugt werden gemäß dem vierten Aspekt die genannten Tumorzellen in der Tumorprobe, die keine Tumorstammzellen sind und ein im Vergleich zu Tumorstammzellen schnelleres Zellteilungsverhalten aufweisen einer selektiven Markierung unterzogen.

Bevorzugt ist die Markierung im Zusammenhang mit dem vierten Aspekt gemäß der Markierung im Zusammenhang mit dem ersten und/oder zweiten Aspekt definiert.

Bevorzugt ist das Mittel im Zusammenhang mit dem vierten Aspekt ein Vektor, der gemäß dem Vektor im Zusammenhang mit dem ersten und/oder zweiten Aspekt definiert ist. Weitere bevorzugte Ausführungsformen des vierten Aspekts ergeben sich aus denen des ersten und/oder zweiten Aspekts. Insofern wird diesbezüglich auf die oben stehenden Ausführungen verwiesen.

In einem weiteren Aspekt betrifft die vorliegende Erfindung ein Verfahren zur Diagnose einer bestimmten Krebsart, umfassend ein hierin offenbartes Verfahren bzw. eine hierin offenbarte Verwendung nach dem ersten, zweiten, dritten und/oder vierten Aspekt, dadurch gekennzeichnet, dass aus dem Vorliegen von bestimmten Tumorstammzelle die Diagnose einer zugehörigen bestimmten Krebsart vorgenommen wird.

In einem weiteren Aspekt betrifft die vorliegende Erfindung ein Verfahren zur Expansion von Tumorstammzellen, umfassend ein hierin offenbartes Verfahren bzw. eine hierin offenbarte Verwendung nach dem ersten, zweiten, dritten und/oder vierten Aspekt, sowie einen weiteren Schritt der Vermehrung der Tumorstammzellen. Die letztgenannten Verfahren beinhalten Verfahren zum Herstellen von Zellkulturen von Tumorstammzellen.

In einem weiteren Aspekt betrifft die vorliegende Erfindung ein Verfahren zur Identifikation von Wirkstoffen gegen Tumorstammzellen, umfassend ein hierin offenbartes Verfahren bzw. eine hierin offenbarte Verwendung nach dem ersten, zweiten, dritten und/oder vierten Aspekt, sowie einen weiteren Schritt des In-Kontakt-Bringens der Tumorstammzellen mit mindestens einem potentiellen Wirkstoff. Bevorzugt werden als potentielle Wirkstoffe sogenannte "Pipeline-Wirkstoffkandidaten" aus dem Gebiet der Onkologie getestet. Solche Wirkstoffkandidaten sind dem Fachmann geläufig. Das Testen kann *in vitro* erfolgen. Alternativ können potentielle Wirkstoffe auch *in vivo-,* beispielsweise über das Applizieren von Tumorstammzellen in immundefiziente Mäuse wie "NOD_SCID-mice" getestet werden. Bevorzugt enthalten Verfahren des letzten Aspekts einen Schritt des Bestimmens, ob der potentielle Wirkstoff als Wirkstoff geeignet ist. Eine Eignung kann sich beispielsweise aus einer Beeinträchtigung des Wachstums der Tumorstammzellen oder einer Beeinträchtigung der Lebensfähigkeit der Tumorstammzellen ergeben.

Das Anwendungsgebiet für Zelltestsysteme ist sehr weit zu fassen. Denkbar sind Anwendungen in der Pharmaindustrie (Wirkstofftestung, Antikörperentwicklung, Zelltherapiestudien) sowie in präklinischen Studien (bildgebende Tumorinitiation über GFPtransduzierte Tumorzellen; Bio-Imageing).

Bevorzugte Ausführungsformen der beschriebenen weiteren Aspekte ergeben sich aus denen des ersten, zweiten, dritten und/oder vierten Aspekts. Insofern wird diesbezüglich auf die oben stehenden Ausführungen verwiesen.

Allgemein ist und/oder beinhaltet in bestimmten Ausführungsformen keiner der hierin offenbarten Gegenstände einen Cytokinstimulation, um Tumorstammzellen zu prolifierieren und in Kolonien zu differenzieren.

Allgemein ist und/oder beinhaltet in bestimmten Ausführungsformen keiner der hierin offenbarten Gegenstände die Messung des aktiven Abtransports einer Markierung (wie z.B. "Hoechst dye" oder Rodamin) mittels P-Glykoprotein aus Zellen.

Allgemein ist und/oder beinhaltet in bestimmten Ausführungsformen keiner der hierin offenbarten Gegenstände ein Verfahren zur spezifischen Markierung (beispielsweise mit PKH26) zum Nachweis von asymmetrischer Zellteilung.

Allgemein ist und/oder beinhaltet in bestimmten Ausführungsformen keiner der hierin offenbarten Gegenstände die Bewertung der Fähigkeit von Tumorstammzellen, in serumfreiem Medium unter Nichtadhäsionsbedingungen sphärenförmige Zellaggregate auszubilden.

Allgemein ist und/oder beinhaltet in bestimmten Ausführungsformen keiner der hierin offenbarten Gegenstände ein Verfahren zur chirurgischen oder therapeutischen Behandlung des menschlichen oder tierischen Körpers.

Allgemein ist und/oder beinhaltet in bestimmten Ausführungsformen keiner der hierin offenbarten Gegenstände ein Diagnostizierverfahren, das am menschlichen oder tierischen Körper vorgenommen wird.

Allgemein ist und/oder beinhaltet in bestimmten Ausführungsformen keiner der hierin offenbarten Gegenstände den Einsatz bzw. die Verwendung totipotenter Stammzellen.

### Beispiele

Die vorliegende Erfindung wird nachfolgend mit Bezugnahme auf nicht einschränkende, erläuternde Beispiele beschrieben werden. Es wird verstanden werden, dass die Erfindung auch jegliche Auswahlen und Kombinationen der im Zuge der Beispiele offenbarten Merkmale vorsieht.

### 1. Gentransfer über retrovirale Vektorsysteme / MoMLV

Im Zusammenhang mit retroviralen Vektorsystemen wird zunächst vollumfänglich auf die Offenbarung im Einleitungsteil Bezug genommen. Dabei wurde u.a. dargelegt, dass beispielsweise Viren / retrovirale Vektorsysteme, die auf dem Moloney murinen Leukämie Virus (MoMLV) basieren, nur sich teilende Zellen transduzieren, da sie über keinen aktiven Transportmechanismus durch die Kemmembran der Zielzelle verfügen.

Zur Herstellung von viralen Vektoren werden Expressionskassetten für das virale Genom und die notwendigen Bestandteile zur Virusproduktion im Fachgebiet oft auf verschiedenen Plasmiden kodiert. Diese Plasmide werden über Transfektion in Zellen eingebracht, in denen dann die Herstellung der Viruspartikel stattfindet. Hierbei wird unterschieden wie viele Plasmide notwendig sind, damit Viruspartikel gebildet werden. Je mehr Funktionen für die Erstellung von Viruspartikel auf verschiedenen Plasmiden verteilt sind, desto sicherer ist das System gegenüber von Rekombinationen, die wieder zu replikationsfähigen Viren führen könnten.

Es wurde ein virales Vektorsystem verwendet, welches auf dem Retrovirus MoMLV basiert (Schuck S et al., 2004). Hierbei werden replikationsinkompetente, sich selbst inaktivierende virale Vektoren erstellt. Die Inaktivierung geschieht durch Promotorinterferenz (Emerman M et al., 1984), da hier die 5'-LTR ein Hybrid darstellt aus einem CMV und einem *mouse sarcoma virus* (MSC) Promotor (CMV/MSCp) und dieser in der 3'-LTR *enhancer region* (U3) deletiert wurde. Das "Shuttle-Plasmid" pRVH1 und seine Derivate (Barton GM und Medzhitov R, 2002; vgl. Abbildung 1) enthalten die notwendigen Elemente zur Prozessierung viraler RNA aus den LTR-Sequenzen, die tRNA-Primerbindungsstelle und das erweiterte Verpackungssignal (Ψ⁺) (Armentano D et al., 1987). Die Env-Region ist deletiert. Die Expression von gag und pol muss entweder auf einem weiteren Vektor kodiert werden oder es wird eine Verpackungszelllinie wie z.B. Phoenixzellen (http://www.stanford.edu/group/nolan/) verwendet, welche die erforderlichen Proteine stabil exprimieren (Abbildung 1).

### 2. Pseudotypsierung der viralen Vektoren

Über eine Pseudotypisierung kann der Zelltropismus eines viralen Vektors verändert werden (Mochizuki, 1998). Der hier genutzte virale Vektor hatte eine Deletion im Bereich seines Hüllproteins, so dass eine Pseudotypisierung über die externe Expression von dem Glykoprotein des Vesikulären Stomatitisvirus (VSV-G) erfolgen konnte. Über VSV-G können nahezu ubiquitär Säugetierzellen infiziert werden (Naldini L et al., 1996). Dabei interagiert das VSV-G mit den in der Zellmembran der Zielzelle vorhandenen Phospholipiden, so dass kein spezifischer Virusrezeptor notwendig ist (Mastromariono P et al., 1987; Yao Y et al., 2003). Der Vektor pHit-G (Fouchier RA et al., 1997) kodiert für VSV-G (Rose JK, 1982). Das Plasmidbackbone pHit (Soneoka Y et al., 1995) ermöglicht dessen Expression über einen CMV Promotor. Durch den *origin of replication* (ori) des simian virus 40 (SV40) wird in Zellen, welche das Large T-Antigen von SV40 enthalten, die Expression erhöht. Die über VSV-G vermittelte Fusion führt zum Eindringen des Virus in die Zielzelle und findet bei einem sauren pH-Wert statt, nachdem die Viruspartikel über Endozytose in die Zelle aufgenommen worden sind. Zusätzlich zur Eigenschaft der Pseudotypisierung wird durch VSV-G auch eine höhere Partikelstabilität der viralen Vektoren erreicht, so dass eine Aufkonzentrierung der viralen Partikel über Ultrazentrifugation möglich wird (Bums JC et al., 1993).

### 3. Kultivierung von eukaryotischen Zellen

Die Wachstumsbedingungen zur Kultivierung von eukaryotischen Zellen im Brutschrank betrugen 37 °C bei einer Luftfeuchtigkeit von 90 % und einem CO₂-Gehal von 5 %. Es wurde nur mit adhärent wachsenden Zellen gearbeitet, zum einen mit primären, aus Gewebe isolierten Zellen und zum anderen mit sekundären, transformierten Zelllinien (vgl. Tabelle 2). Das Passagieren der Zellen erfolgte nach der Abnahme des Kultivierungsmediums und einem Waschschritt mit PBS mit Trypsin / EDTA. Trypsin spaltet Peptidbindungen, so dass die Adhärenz der Zellen verloren geht. Dafür wurde Trypsin auf die adhärenten Zellen gegeben, so dass diese gerade eben bedeckt waren. Anschließend wurden die Zellen mit dem Trypsin bei 37 °C inkubiert. Dabei wurden immer kontrolliert, ob die Zellen beginnen, sich von der Wachstumsoberfläche abzulösen. Sobald dies geschah, wurde der enzymatische Prozess sofort durch die Zugabe von serumhaltigem Kultivierungsmedium (vgl. Tabelle 1) gestoppt. Der Zustand der Zellen wurde anhand ihrer Morphologie regelmäßig unter dem Stereomikroskop kontrolliert. Dabei wurde darauf geachtet, dass die Zellen niemals mehr als 90 % Konfluenz erreicht hatten, bevor sie passagiert wurden. Werden die Zellen erst bei einer höheren Konfluenz passagiert, so kann es zu einer Veränderung der Eigenschaften der Zellen kommen, wie z.B. dass sie bei der nächste Passage keine Einzelschicht (hierin auch als "Monolayer" bezeichnet) mehr ausbilden, sondern in Zellaggregaten wachsen. Beim Passagieren wurden jeweils 1/10 der Zellen nach dem Passagieren weitergeführt.

**Tabelle 1: Kultivierungsmedien der verwendeten eukaryotischen Zellen**

| **Zellen** | **Medium** | **Zusätze** |
|---|---|---|
| 293T / Phoenix | DMEM w. Glutamax 1 | 10 % FKS |

**Tabelle 2: Zelllinien**

| **Zelllinien** | **Beschreibung** |
|---|---|
| 293T | Humane Nierenepithelzellen, die mit Adenovirus Typ 5 (Ad5) transformiert wurden. Expression des großen T-Antigens von SV40 (vgl. DuBridge, 1987), beispielsweise kommerziell erhältlich als "CRL-11268" via ATCC |
| Phoenix | Ursprung 293T-Zellen, stabil transduziert mit gag-pol Verpackungsplasmid basierend auf MoMLV (vgl. z.B. http://www.stanford.edu/group/nolan/) |

### 4. Standardprotokoll Transfektion

Es wurde mit einem Verhältnis von 1,5 µ1 Lipofectamine 2000 zu 1 µg DNA pro 2 cm² Wachstumsfläche der Zellen gearbeitet. Je Ansatz wurde 1 µg DNA in 50 µl OptiMEM gegeben ohne einen weiteren Inkubationsschritt. Je 1,5 µl Lipofectamine 2000 wurde in ebenfalls 50 µl OptiMEM gegeben und anschließend für 5 min bei RT inkubiert. Dieser Zeitraum wurde immer exakt eingehalten. Danach erfolgte die Zugabe der DNA-Lösung zum Lipofectamine-2000-Lösung und beides wurde für 20 min bei RT inkubiert. Die zu transfizierenden Zellen wurden 24 h vorher in der entsprechenden Zellzahl ausplattiert, so dass zum Zeitpunkt der Transfektion 90% optische Konfluenz erreicht war. Das Kultivierungsmedium wurde abgenommen und die Zellen einmal mit 1x PBS gewaschen. Anschließend wurde der DNA-Lipofectamine-2000-Komplex auf die Zellen gegeben und äquivalent wie bei der Transfektion mittels PEI zusätzlich noch 400 µl OptiMEM hinzugegeben, um ein Austrocknen der Zellen zu vermeiden. Nach 4 bis 6 h erfolgte auch hier ein Mediumwechsel mit frischem Kultivierungsmedium.

### 5. Herstellung von viralen Vektoren

Zur Herstellung von viralen Vektoren wurden die notwendigen Plasmide zur Produktion von viralen Partikeln in Zellen transfiziert. Für die Herstellung von retroviralen Partikeln basierend auf MoMLV wurden Phoenix-Zellen, die stabil die Proteine der gag-pol-Region von MoMLV exprimieren, verwendet. Bei diesen viralen Vektorsystemen wurde mit VSV-G pseudotypisiert. Die verwendeten Plasmide und ihr Verhältnis bei der Transfektion sind der Tabelle 3 zu entnehmen. Die Transfektion erfolgte entweder mittels Polyethylenimin (PEI) oder Lipofectamine 2000. Dabei zählte der Beginn der Transfektion als Stunde null. Nach 4 bis 6 h Inkubationszeit der Transfektion wurde ein Mediumwechsel durchgeführt mit einem weniger nährstoffhaltigem Medium, damit die Zellen langsamer proliferieren und eine erhöhte Menge an viralen Partikeln gebildet werden konnte. Hierfür wurde jeweils mit DMEM mit Glutamax I und einem niedrigen Glukosegehalt (1000 mg/1) mit 5 % FKS-Zusatz gearbeitet. Pro 2 cm² Wachstumsfläche wurden immer 0,4 ml Medium verwendet, so dass je nach Transfektionsmethode immer ein vergleichbarer Virustiter gebildet werden konnte. Nach 48 h p.t. wurde der Überstand mit den freigesetzten viralen Partikeln abgenommen und über einen Zellulose-Acetat-Filter mit 0,45 µm Poren filtriert.

**Tabelle 3: Verhältnis der eingesetzten Plasmidmenge zur Produktion von viralen Vektoren.**

| **Virussystem** | ***Shuttle*-Plasmid** | **pHit-G** | **Gag-pol-Plasmid** |
|---|---|---|---|
| MoMLV (293T) | 2,2 | 1 | 1 |
| MoMLV (Phoenix) | ∼1,8 | 1 | (in den Phoenix-Zellen) |

### 6. Transduktion von Zellen mit viralen Vektoren / Vektorsystemen

Zur Transduktion von Zellen wurden diese 24 h vorher in Zellkulturplatten ausgesät, so dass sie zum Zeitpunkt der Transduktion zwischen 50 bis max. 90 % optische Konfluenz aufwiesen. Dem Überstand mit den viralen Vektoren wurde Polybren mit einer Endkonzentration von 4µg/ml hinzugegeben. Die Zielzellen wurden einmal mit 1x PBS gewaschen und anschließend der virushaltigen Überstand dazugegeben. Wenn eine Verdünnung der viralen Vektoren notwendig war, wurde dafür OptiMEM verwendet. Die Zellen wurden dann bei 22° C und 200x g für 90 min zentrifugiert. Das Polybren kann mit den viralen Vektoren interagieren, so dass diese schon bei einer geringen Beschleunigung von 200x g sedimentieren und damit direkt auf der Oberfläche der Zielzellen auftreffen. Nach dem Zentrifugationsschritt erfolgt ein sofortiger Mediumwechsel mit frischem Kultivierungsmedium. Die Kontrolle der Transduktionseffizienz erfolgte jeweils mit den EGFP-exprimierenden viralen Vektoren 48 h nach der Transduktion.

Mit dem nun vorliegenden viralen Vektorsystem, basierend auf MoMLV, konnten nun eukaryotische Zellen, beispielsweise primäre humane Zellen transduziert werden. Auf MoMLV-basierende virale Vektorsysteme konnten beispielsweise primäre Fibroblasten und humane Tumorzellen (Mamma-, Ovarial-, Pankreas-, Prostata-, Urothelkarzinom und Renal-Cell-Carcinoma) transduzieren. Als Kontrollzellen wurden bei jedem Ansatz 293T-Zellen transduziert. Die Expression des Reporters EGFP kann 24 h nach Transduktion schon leicht zu erkennen sein, aber erst nach mindestens 48 h ist eine Auswertung anhand der Fluoreszenz möglich.

### 7. Kultivierung GFP-transduzierter Tumorzelllinien

Für eine nachfolgende Zuweisung der Stammzelleigenschaften von Subpopulationen innerhalb einer Tumoreinzelzellsuspension, wurden die viral transduzierten Zelllinien in RPMI + Glutamax + 10% FKS expandiert. Die Wachstumsbedingungen zur Kultivierung der verschiedenen humanen Tumorzelllinien (vgl. Tabelle 4) betrugen 37 °C bei einer Luftfeuchtigkeit von 90% und einem CO₂-Gehal von 5%. Das Passagieren der Zellen erfolgte nach der Abnahme des Kultivierungsmediums und einem Waschschritt mit PBS mit Trypsin /EDTA. Trypsin spaltet Peptidbindungen und das EDTA "fängt" als sogenannter Chelatbildner aller Ca⁺⁺-Ionen ein, so dass die kalziumabhängige Adhärenz der Zellen verloren geht. Dafür wurde Trypsin auf die adhärenten Zellen gegeben, so dass diese gerade eben bedeckt waren. Anschließend wurden die Zellen mit dem Trypsin bei 37 °C inkubiert. Dabei wurden immer kontrolliert, ob die Zellen beginnen, sich von der Wachstumsoberfläche abzulösen. Sobald dies geschah, wurde der enzymatische Prozess sofort durch die Zugabe von serumhaltigem Kultivierungsmedium gestoppt. Der Zustand der Zellen wurde anhand ihrer Morphologie regelmäßig unter dem Stereomikroskop kontrolliert. Dabei wurde darauf geachtet, dass die Zellen niemals mehr als 90 % Konfluenz erreicht hatten, bevor sie passagiert wurden. Werden die Zellen erst bei einer höheren Konfluenz passagiert, so kann es zu einer Veränderung der Eigenschaften der Zellen kommen, wie z.B. dass sie bei der nächste Passage keinen Monolayer mehr ausbilden, sondern in Zellaggregaten wachsen. Beim Passagieren wurden jeweils 1/10 der Zellen nach dem Passagieren weitergeführt.

**Tabelle 4: Repräsentative Tumorzelllinien**

| **Zelllinien** | **Entität** | **Primär** | **Linie** | **Beschreibung der Transduktion** |
|---|---|---|---|---|
| MT_012 | Mamma | x | | Ursprung 293T-Zellen, stabil transduziert mit gag-pol Verpackungsplasmid basierend auf MoMLV (http://www.stanford.edu/group/nolan/) |
| OV_001 | Ovar | x | | |
| RCC_001 | Ren | x | | |
| RCC_003 | Ren | x | | |
| DAN-G | Pankreas | | x | |

Nach dem Ernten der Zellen erfolgte die Markierung von spezifischen Stammzelloberflächenantigen mittels stammzellspezifischer Antikörper, die jeweils mit einem bestimmten Fluoreszenzfarbstoff konjugiert sind.

### 8. Standardprotokoll zur Immunphänotypisierung ruhender Tumorzellen in der Durchflusszytometrie (FACS-Analyse)

Nach der Kultivierung (bis max. 80% Konfluenz) wurden die Zellen geerntet, zweimal mit 1x PBS gewaschen und zu je 5x 10⁵ Zellen in 200µl pro sogenanntes FACS-Röhrchen aliquotiert. Nun wurden 4µl eines direktmarkierten monoklonalen Antikörpers; mAK (in jeweiliger Gebrauchsverdünnung) bestimmter Spezifität (z.B. CD44, oder CD24, oder CD133) und unterschiedlicher Fluoreszenzkonjugation (z.B. PE*, oder APC, oder PC5) bzw. PBS (als Negativkontrolle) hinzugefügt und alle Ansätze für 45 min bei 4 °C inkubiert (vgl. Tabelle 5). Danach wurden die Zellen dreimal mit 10% Gelafusal (4% flüssige Gelatine in Ringer-Acetat) in 1x PBS gewaschen (2 min, 1200 rpm). Abschließend wurden die Zellen bei Bedarf mit 1%iger Formaldehydlösung fixiert (wenn die Messung zeitversetzt stattfand). Die Detektion einer Tumorzellfraktion mit Stammzelleigenschaften, in der jeweiligen Probe, erfolgte mit Hilfe der Durchflusszytometrie am FACS (FC500; Beckman Coulter).

**Tabelle 5 Antikörper (direktmarkiert)**

| **Antikörper** | **Konjugation** | **Beschreibung** |
|---|---|---|
| CD44 | Phycoerythrin (PE) | Hierzu wird vollumfänglich auf die oben stehende Offenbarung Bezug genommen. |
| CD133 | Allophycocyanin (APC) | Hierzu wird vollumfänglich auf die oben stehende Offenbarung Bezug genommen. |
| CD24 | Allophycocyanin (APC) | Hierzu wird vollumfänglich auf die oben stehende Offenbarung Bezug genommen. |
| CD326 | Phycoerythrin (PE) | Hierzu wird vollumfänglich auf die oben stehende Offenbarung Bezug genommen. |
| CA_09 | Phycoerythrin (PE) | Tumormarker des hellzelligen Nierenzellkarzinoms, hierzu wird außerdem vollumfänglich auf die oben stehende Offenbarung Bezug genommen |
| CXCR4 | Allophycocyanin (APC) | Tumormarker des metastasierenden Pankreaskarzinoms |

### 9. Identifikation und Separation von Tumorstammzellen

Nach erfolgreicher retroviraler Transduktion und dem Einbringen des GFP-Reporters in die sich schnell teilenden Zellen (vgl. Abbildung 2), bleiben alle ruhenden Zellen (Tumorzellen mit Stammzelleigenschaften) GFP-negativ und können in der Durchflusszytometrie auch so dargestellt werden. Dazu wurden im Anschluss die jeweiligen Einzelzellsuspensionen der verschiedenen Tumorentitäten, entsprechend der oben beschriebenen Prozedur, einer Antikörperfärbung unterzogen. Im Verlauf dieser Antikörperfärbungen, zur Negativselektion ruhender GFP-negativer Tumorzellen, wurde auf die Konjugation mit dem Fluoreszenzfarbstoff Fluorescein (Fluoresceinisothiocyanat [FITC]) verzichtet, da der Fluoreszenzkanal FL_1 durch die konstitutive Expression des GFP in den transduzierten Zellen belegt war.

Dementsprechend wurde eine Gating-Strategie entworfen, die es ermöglichte die GFP-negativen Zellen mit der Expression bekannter Stammzell-, Adhäsions- (CD44, CD24, CD133) und Tumormarker (CD326, CA_09, CXCR4) zu korrelieren. In diesem Zusammenhang detektierten die vorliegenden Erfinder, über eine Dotplot-Darstellung, den Granulationsgrad der Zellen im Seitwärts-Scatter (SS) gegen die Fluoreszenzintensität der Zellen im Kanal FL-1 für das GFP, bei ca. 520nm. In dieser Einstellung konnten alle vitalen Zellen, die GFP-negativ waren, über den Bereich des Seitwärts-Scatters in einer Region zusammengefasst werden ("gated"). Diese Zellen sind eine Population ruhender Zellen, da der MoMLV-basierte Vektor nur sich teilende Zellen infiziert. In den folgenden Dotplot-Darstellungen, wurden nun zwei verschiedene Fluoreszenzen - welche immer zwei Antikörperspezifitäten repräsentierten - gegen einander aufgetragen. Vorzugsweise wurde der Stammzellmarker CD44 im Fluoreszenzkanal 2 (FL-2) über das Phycoerythrin gemessen, während der CD133-Marker, für hämatopoetische Stammzellen, im Kanal 4 (FL-4) über das Allophycocyanin detektiert wurde. All diesen Dotplots wurde nun die eingegrenzte Region GFP-negativer Zellen zugewiesen ("gated"). Dies bedeutet, dass nur die Zellen dieser GFP-negativen Region überhaupt in den folgenden Plots zur Darstellung gelangen. Mit anderen Worten, nur die Oberflächenexpression der Zellen in der GFP-Negativregion wird hierbei detektiert (Gating-Strategie). Die folgende Darstellung repräsentiert die prinzipielle Struktur des Assays zur Negativselektion von ruhenden Tumorzellen mit Stammzelleigenschaften am Beispiel der kommerziellen Pankreaskarzinomzelllinie DAN-G. Es konnte gezeigt werden, dass die ruhenden GFP-negativen Zellen der DAN-G-Linie eine Biomarkersignatur für CD44+/CD24- aufweisen. Damit gehört diese Zelllinie zu den nicht-metastasierenden Karzinomen dieser Entität (vgl. Abbildung 3). Die Eliminierung von toten oder apoptotischen Zellen (Anexin V- und 7AAD-Analyse) erfolgt vor einer solchen Zuweisung ebenso über eine Fluoreszenzzellsortierung. Darüber hinaus gibt es gleichsam postmitotische Bindegewebszellen, die auch als "ruhend" erscheinen können, sich aber dann natürlich nicht mit Stamm- oder Tumorzelleigenschaften darstellen lassen. In diesem Zusammenhang identifizieren die vorliegenden Erfinder jeweils die entsprechende Biomarker-Signatur für die Tumorstammzellen der vorliegenden Probe. Die so gewonnene Tumorstammzellfraktion ist von einer sehr hohen Signifikanz gekennzeichnet und wird dann einer weiteren Validierung (Anwachsen im Tier, im Vergleich zu den depletierten, sich schnell teilenden und nicht-tumorigenen Tumorzellen [diese dürfen nicht anwachsen]) und Charakterisierung (RNA-Chiptechnologie [Top-Kandidaten für Überexpression], Western Blot [Expression von Genprodukten der Stammzell-Pathways]) zugeführt. Insofern stellt die vorliegende Technologie nicht allein auf die Erkennung über die bekannten Stammzelloberflächenmarker ab, sondern kombiniert diese nach der fraktionierten Anreicherung aller ruhenden Zellen der Tumorprobe. Die Darstellungen zur Identifikation ruhender Tumorzellen mit Stammzelleigenschaften weiterer Entitäten sind in Abbildung 4 zusammengefasst. Somit wird hier eine beispielhafte Biomarker-Signatur für Tumorstammzellen dargestellt, die durch Ein- bzw. Ausschluss weitere Tumormarker erweitert werden kann.

### Referenzen

Alam J und Cook JL, 1990, Anal Biochem., 188:245-254
Al-Hajj M et al., 2003, Proc. Natl. Acad. Sci. USA, 100:3983-3988
Armentano D, et al., 1987, J Virol., 1987 May;61(5):1647-50.
Barton GM und Medzhitov R, 2002, Proc. Natl. Acad. Sci. USA, 99:14943-14945 Bui MH et al., 2003, Clin Cancer Res., 9:802-811
Bums JC et al., 1993, Proc Natl Acad Sci USA, 90:8033-8037
Chalfie M et al., 1994, Science, 263:802-805
DuBridge RB et al.,1987, Mol Cell Biol., 7:379-387
Emerman M et al., 1984, Cell, 39:449-467
Fouchier RA et al., 1997, EMBO J, 16:4531-4539
Fouchier RA und Malim MH, 1999, Adv Virus Res., 52:275-299
Ivanov S et al., 2001, Am JPathol.,158:905-919
Kim HL et al., 2005, J Urol., 173:1496-1501
Lam JS et al., 2005, Urology., 66(5 Suppl):1-9 [Review]
Leibovich BC et al., 2003, Cancer, 97:1663-1671.
Leibovich BC et al., 2007, J Clin Oncol., 25:4757-4764
Levy JA et al., 1995, The Retroviridae, Plenum Press, New York
Liao SY et al., 1997, Cancer Res., 57:2827-2831
Mastromariono P et al., 1987, J Gen Virol, 68:2359-2369
Mochizuki H et al., 1998, J Virol, 72: 8873 8883
Mann R et al., 1983, Cell, 33: 153-159
Murakami Y et al., 1999, Brit J Uro Int., 83:743-747
Naldini L et al., 1996, Proc Natl Acad Sci USA, 93: 11382 -11388
Naldini L et al., 1996, Science, 272: 263-267
Oosterwwijk E et al.; 1986, Int J Cancer, 38:489-494.
Patard JJ et al., 2008, Int J Cancer, 123:395-400.
Prasher DC et al., 1992, Gene, 111:229-233
Roe T et al., 1993, EMBO J, 12:2099-2108
Rose JK et al., 1982, Cell, 30:753-762
Sandlund J et al., 2007, Brit J Uro Int., 100:556-560
Schuck S et al., 2004, Proc Natl Acad Sci USA, 101:4912-4917
Soneoka Y et al., 1995, Nucleic Acids Research, 23:628-633
Yao Y et al., 2003, Virology, 310:319-332

## Patentansprüche

1. Verfahren zur Detektion und/oder Identifikation von Tumorstammzellen in einer Tumorprobe, umfassend Auswählen von Tumorstammzellen basierend auf ihrem Zellteilungsverhalten,
**dadurch gekennzeichnet, dass** Tumorzellen in der Tumorprobe, die keine Tumorstammzellen sind und ein im Vergleich zu Tumorstammzellen schnelleres Zellteilungsverhalten aufweisen, einer Markierung unterzogen werden.

2. Verfahren zur Gewinnung von Tumorstammzellen aus einer Tumorprobe, umfassend Auswählen von Tumorstammzellen basierend auf ihrem Zellteilungsverhalten, **dadurch gekennzeichnet, dass** Tumorzellen in der Tumorprobe, die keine Tumorstammzellen sind und ein im Vergleich zu Tumorstammzellen schnelleres Zellteilungsverhalten aufweisen, einer Markierung unterzogen werden.

3. Verfahren nach Anspruch 1 oder 2, wobei das Verfahren ein *Ex-vivo*-Verfahren ist oder wobei das Verfahren ein *In-vitro*-Verfahren ist.

4. Verfahren nach einem beliebigen der vorangehenden Ansprüche, wobei das Auswählen der Tumorstammzellen eine Negativauswahl darstellt,
insbesondere wobei die genannten Tumorzellen in der Tumorprobe, die keine Tumorstammzellen sind und ein im Vergleich zu Tumorstammzellen schnelleres Zellteilungsverhalten aufweisen, einer selektiven Markierung unterzogen werden.

5. Verfahren nach Anspruch 4, wobei die Markierung über einen Vektor, bevorzugt viralen Vektor, stärker bevorzugt retroviralen Vektor, in die genannten Tumorzellen in der Tumorprobe, die ein im Vergleich zu Tumorstammzellen schnelleres Zellteilungsverhalten aufweisen, eingeführt wird,
insbesondere wobei der Vektor nur von sich teilenden Zellen aufgenommen wird,
im Besonderen wobei der Vektor replikationsinkompetent ist, bevorzugt wobei der Vektor auf dem Retrovirus MoMLV basiert, insbesondere wobei der Vektor unter Verwendung von Phoenix-Zellen hergestellt wurde.

6. Verfahren nach einem beliebigen der Ansprüche 4 und 5, wobei die Markierung eine Fluoreszenzmarkierung, bevorzugt eine GFP-Markierung, ist,
insbesondere wobei die Fluoreszenzmarkierung über einen Vektor in die genannten Tumorzellen in der Tumorprobe, die ein im Vergleich zu Tumorstammzellen schnelleres Zellteilungsverhalten aufweisen, eingeführt wird, wobei der Vektor bevorzugt wie in Anspruch 5 definiert ist,
im Besonderen wobei das Einführen mittels Transduktion erfolgt,
insbesondere wobei der Vektor aus in Phönixzellen produzierten Viruspartikeln besteht, mit denen die Tumorzellen infiziert werden.

7. Verfahren nach einem beliebigen der vorangehenden Ansprüche, wobei die Detektion und/oder Identifikation bzw. die Gewinnung weiterhin Bestimmen von mindestens einem Oberflächenmarker auf den Tumorstammzellen umfasst,
insbesondere wobei eine Gruppe von Oberflächenmarkern, umfassend Stammzellmarker, Adhäsionsmarker, und/oder Tumormarker bestimmt wird, und/oder wobei der/die Oberflächenmarker ausgewählt ist/sind aus der Gruppe, bestehend aus CA_09, CD10, CD11, CD24, CD34, CD38, CD44, CD45, CD73, CD133, CD326, CXCR4 und EpCAM.

8. Verfahren nach einem beliebigen der vorangehenden Ansprüche, wobei der mindestens eine Oberflächenmarker oder die Gruppe von Oberflächenmarkern unter Verwendung von mindestens einem markierten Antikörper bestimmt wird, insbesondere wobei der mindestens eine markierte Antikörper eine Fluoreszenzmarkierung trägt,
im Besonderen wobei für verschiedene Oberflächenmarker verschiedene Fluoreszenzmarkierungen, bevorzugt ausgewählt aus Flurescein, Phycoerythrin und Allophycocyanin, verwendet werden,
bevorzugt wobei sich die Fluoreszenzmarkierung(en) von der in den Ansprüchen 4-6 definierten Markierung unterscheidet/n.

9. Verfahren nach einem beliebigen der vorangehenden Ansprüche, umfassend das Herstellen einer Einzelzellsuspension aus der Tumorprobe,
insbesondere wobei das Auswählen der Tumorstammzellen aus einer Einzelzellsuspension erfolgt,
im Besonderen wobei das Verfahren die Verwendung von Durchflusszytometrie, insbesondere von FACS-Analyse umfasst.

10. Verfahren nach einem beliebigen der vorangehenden Ansprüche, umfassend einen Kontrollschritt, um das Auswählen apoptotischer und/oder nekrotischer Zellen zu vermeiden.

11. Verfahren nach einem beliebigen der vorangehenden Ansprüche, wobei die Tumorstammzellen ausgewählt sind aus der Gruppe, bestehend aus Tumorstammzellen aus Mammakarzinom, Ovarialkarzinom, Pankreaskarzinom, Prostatakarzinom, Urothelkarzinom und Nierenzellkarzinom.

12. Tumorstammzellen, identifiziert nach einem Verfahren der Ansprüche 1 bis 11, und erhältlich nach einem Verfahren der Ansprüche 2 bis 11, zur Verwendung in der Medizin.

13. Verwendung eines Mittels, geeignet zum Auswählen von Tumorstammzellen basierend auf ihrem Zellteilungsverhalten,
i) bei der Detektion und/oder Identifikation von Tumorstammzellen in einer Tumorprobe
und/oder
ii) bei der Gewinnung von Tumorstammzellen aus einer Tumorprobe,
**dadurch gekennzeichnet, dass** das Mittel die Markierung von Tumorzellen in der Tumorprobe, die keine Tumorstammzellen sind und ein im Vergleich zu Tumorstammzellen schnelleres Zellteilungsverhalten aufweisen, ermöglicht.

14. Verwendung nach Anspruch 13, wobei das Auswählen der Tumorstammzellen eine Negativauswahl darstellt,
insbesondere wobei die genannten Tumorzellen in der Tumorprobe die keine Tumorstammzellen sind und ein im Vergleich zu Tumorstammzellen schnelleres Zellteilungsverhalten aufweisen einer selektiven Markierung unterzogen werden,
im Besonderen wobei die Markierung gemäß Anspruch 6 definiert ist und/oder wobei das Mittel ein gemäß Anspruch 5 definierter Vektor ist.

15. Verwendung nach einem der Ansprüche 13 und 14, weiter gekennzeichnet gemäß einem der Ansprüche 3 bis 11.
